Europäisches Patentamt

European Patent Office

Office européen des brevets

(11) Publication number: **0 252 380 B1**

# EUROPEAN PATENT SPECIFICATION

(45) Date of publication of patent specification: **15.04.92**

(21) Application number: **87109199.7**

(22) Date of filing: **26.06.87**

(51) Int. Cl.⁵: **C07G 11/00**, C12P 13/00, A23K 1/17

The file contains technical information submitted after the application was filed and not included in this specification

(54) **Antibiotic LL-E19020 alpha and beta.**

(30) Priority: **30.06.86 US 880230**
**30.06.86 US 880239**
**30.06.86 US 880608**
**30.06.86 US 880229**

(43) Date of publication of application:
**13.01.88 Bulletin 88/02**

(45) Publication of the grant of the patent:
**15.04.92 Bulletin 92/16**

(84) Designated Contracting States:
**AT BE CH DE ES FR GB GR IT LI LU NL SE**

(73) Proprietor: **AMERICAN CYANAMID COMPANY**
**1937 West Main Street P.O. Box 60**
**Stamford Connecticut 06904-0060(US)**

(72) Inventor: **Carter, Guy Thomas**
**8 Prairie Avenue**
**Suffern New York 10901(US)**
Inventor: **Greenstein, Michael**
**45 Lorna Lane**
**Suffern New York 10901(US)**
Inventor: **Goodman Joseph Jacob**
**134 Grotke Road**

**Spring Valley New York 10977(US)**
Inventor: **Borders, Donald Bruce**
**13 Heatherhill Lane**
**Suffern New York 10901(US)**
Inventor: **Maiese, William Michael**
**891 Sherwood Road**
**Bridgewater New Jersey 08807(US)**
Inventor: **Testa, Raymond Thomas**
**55 Rockledge Place**
**Cedar Grove New Jersey 07009(US)**
Inventor: **Wood, Irwin Boyden**
**211 Elm Avenue**
**Morrisville Pennsylvania 19067(US)**
Inventor: **Doscher, Mary Eulers**
**147 Gary Drive**
**Trenton New Jersey 08690(US)**
Inventor: **Kantor, Sidney**
**4A Martin Van Buren Drive**
**Cranbury New Jersey 08513(US)**
Inventor: **Kennett, Robert Lee, Jr.**
**Box 203, RD 1**
**Lambertville New Jersey(US)**

(74) Representative: **Wächtershäuser, Günter, Dr.**
**Tal 29**
**W-8000 München 2(DE)**

**Description**

SUMMARY OF THE INVENTION

This invention relates to new antibacterial agents designated LL-E19020 alpha and LL-E19020 beta, to their production by fermentation, to methods for their recovery and concentration from crude solutions and to processes for their purification. The present invention includes within its scope the agents in dilute form, as crude concentrates, as a complex of all components, in pure form as individual components and a novel strain of Streptomyces.

This invention also relates to methods and compositions for increasing the growth rate of meat producing animals, improving the efficiency of feed utilization thereby and enhancing lactation in lactating ruminant animals using LL-E19020 alpha, LL-E19020 beta or a physiologically acceptable salt thereof.

The compounds of this invention are useful for the treatment of both monogastric and ruminant animals. Moreover, said compounds are particularly effective for improving feed efficiency and inducing weight gains in cattle, sheep, swine, goats, rabbits, horses and poultry.

The present invention relates to methods and compositions effective for preventing, treating, controlling or ameliorating, protozoal infections in warm-blooded animals, particularly in poultry, cattle, sheep, swine and goats and in companion animals such as dogs and rabbits using LL-E19020 alpha, LL-E19020 beta and the pharmaceutically and pharmacologically acceptable salts of said antibiotics.

The above antibiotics are also effective for controlling protozoan infections caused by Eimeria and Babesia species in cattle, sheep, swine, chickens, turkeys, ducks, geese and dogs.

It is also anticipated that the antibiotic compositions of this invention will prove to be effective for controlling malaria, sarcosporidiosis and toxoplasmosis in warm-blooded animals since the causative agents for these diseases are protozoan infections biologically related to Eimeria and Babesia.

The present invention provides a composition and method for controlling or preventing helminth and nematode infestation of warm-blooded animals administering thereto, a prophylactically, pharmaceutically or therapeutically, effective amount of an antibiotic selected from LL-E19020 alpha, LL-E19020 beta or a pharmaceutically or pharmacologically acceptable salt thereof. More particularly, this invention provides a method of preventing, controlling or treating helminth and nematode infestations in humans and a wide variety of animals including farm, companion, circus and zoo animals, and it is especially useful and effective for controlling or preventing helminth and nematode infestations in cattle, sheep, swine, horses, goats, rabbits, poultry, dogs, cats and the like.

BRIEF DESCRIPTION OF THE DRAWINGS

Fig. I shows ultraviolet absorption spectra of LL-E19020 alpha.
Fig. II shows an infrared absorption spectrum of LL-E19020 alpha.
Fig. III shows a proton nuclear magnetic resonance spectrum of LL-E19020 alpha.
Fig. IV shows a carbon-13 nuclear magnetic resonance spectrum of LL-E19020 alpha.
Fig. V shows ultraviolet absorption spectra of LL-E19020 beta.
Fig. VI shows an infrared absorption spectrum of LL-E19020 beta.
Fig. VII shows a proton nuclear magnetic resonance spectrum of LL-E19020 beta.
Fig. VIII shows a carbon-13 nuclear magnetic resonance spectrum of LL-E19020 beta.
Fig. IX shows the effect of LL-E19020 alpha on the growth of B. bigemina.

DESCRIPTION OF THE PREFERRED EMBODIMENTS

The structures of antibiotics LL-E19020 alpha and beta have not been elucidated but they are described below in conjuction with their physico-chemical characteristics:

The physico-chemical characteristics of LL-E19020 alpha are as follows:

LL-E19020 alpha

a) Approximate elemental analysis: C 62.73; H 7.60; N 1.00; O 28.67 (by diference);
b) Molecular weight: 1225 (FABMS);
c) Specific rotation: $[alpha]_D^{26}$ = 0 (C 0.385, methanol);
d) Ultraviolet absorption spectra: as shown in Figure I

$$UV^{CH_3OH}_{MAX} = \begin{array}{l} 233nm \ (\epsilon \ 49,800) \\ 290nm \ (\epsilon \ 36,600) \end{array}$$

$$UV^{0.1N \ HCl}_{MAX} = \begin{array}{l} 234nm \ (\epsilon \ 51,500) \\ 300nm \ (\epsilon \ 38,900) \end{array}$$

$$UV^{0.1N \ NaOH}_{MAX} = \begin{array}{l} 217nm \ (\epsilon \ 82,700) \\ 290nm \ (\epsilon \ 45,900) \end{array}$$

e) Infrared absorption spectrum: as shown in Figure II (KBr disc): 3420, 2970, 2925, 1717, 1695, 1647, 1617, 1525, 1445, 1365, 1092, 1018 cm$^{-1}$;

f) Proton nuclear magnetic resonance spectrum: as shown in Figure III (300 MHz, CDCl$_3$);

g) Carbon-13-nuclear magnetic resonance spectrum: as shown in Figure IV (75 MHz, CDCl$_3$, ppm downfield from TMS), significant peaks as listed below:

| | | | | | |
|---|---|---|---|---|---|
| 173.3 | 129.0 | 97.3 | 74.2 | 55.4 | 17.2 |
| 171.4 | 128.6(2x) | 97.0 | 72.0 | 49.8 | 17.0 |
| 170.1 | 128.43 | 89.2 | 71.9 | 41.8(2x) | 14.8 |
| 145.7 | 128.38 | 83.3 | 69.1 | 39.8 | 13.5 |
| 140.3 | 128.1(2x) | 81.6 | 67.5 | 39.1 | 10.8 |
| 137.0 | 127.5 | 77.6 | 66.4 | 38.8 | 10.0 |
| 134.4 | 127.1 | 77.0 | 66.1 | 32.9 | |
| 133.9 | 126.3 | 76.4 | 63.5 | 31.0 | |
| 132.0 | 120.8 | 74.6 | 56.5 | 29.9 | |
| 130.1 | 100.6 | 74.5 | 56.0 | 23.8 | |
| 129.5(2x) | 99.0 | 74.4 | 55.6 | 18.1 | |
| 2x = two overlapping signals | | | | | |

LL-E19020 beta

a) Approximate elemental analysis: C 63.33; H 7.72; N 1.16; O 27.79 (by difference);

b) Molecular weight: 1225 (FABMS);

c) Specific rotation: $[alpha]_D^{26}$ = -17±2(C 0.455, methanol);

d) Ultraviolet absorption spectra: as shown in Figure V

$$UV^{CH_3OH}_{MAX} = \begin{array}{l} 233nm \ (\epsilon \ 47,000) \\ 290nm \ (\epsilon \ 34,100) \end{array}$$

$$UV^{0.1N \ HCl}_{MAX} = \begin{array}{l} 234nm \ (\epsilon \ 46,000) \\ 301nm \ (\epsilon \ 32,800) \end{array}$$

$$UV^{0.1N \ NaOH}_{MAX} = \begin{array}{l} 217nm \ (\epsilon \ 77,800) \\ 290nm \ (\epsilon \ 39,700) \end{array}$$

e) Infrared absorption spectrum: as shown in Figure VI (KBr disc): 3430, 2970, 2930, 1712, 1648, 1620, 1543, 1454, 1367, 1265 1098, 1020, 980 cm$^{-1}$;

f) Proton nuclear magnetic resonance spectrum: as shown in Figure VII (300 MHZ, CDCl$_3$);

g) Carbon-13 nuclear magnetic resonance spectrum, as shown in Figure VIII (75 MHz, $CDCl_3$, ppm downfield TMS), significant peaks as listed below:

| | | |
|---|---|---|
| 173.6 | 99.0 | 55.4 |
| 170.6 | 98.4 | 49.6 |
| 170.0 | 97.2 | 41.6(2x) |
| 145.6 | 89.2 | 39.8 |
| 140.2 | 83.3 | 39.1 |
| 136.7 | 81.6 | 38.0 |
| 134.4 | 77.6 | 32.9 |
| 133.9 | 77.5 | 31.1 |
| 132.0 | 76.2 | 29.9 |
| 130.1 | 75.5 | 23.7 |
| 129.1(2x) | 74.6 | 18.1 |
| 128.9 | 74.5 | 17.2 |
| 128.6(2x) | 74.2(2x) | 17.0 |
| 128.5 | 69.1 | 16.2 |
| 128.4 | 68.9 | 13.5 |
| 128.3 | 67.5 | 10.8 |
| 128.2 | 66.6 | 10.0 |
| 127.8 | 66.1 | |
| 127.2 | 64.1 | |
| 126.5 | 56.5 | |
| 120.9 | 56.0 | |
| 100.6 | 55.6 | |
| 2x = two overlapping signals | | |

The antibiotics of this invention, LL-E19020 alpha and LL-E19020 beta, are formed during the cultivation under controlled conditions of a new strain of Streptomyces lydicus ssp. tanzanius.

This microorganism is maintained in the culture collection of the Medical Research Division, American Cyanamid Company, Pearl River, NY as culture number LL-E19020. A viable culture of this new microorganism has been deposited with the Patent Culture Collection Laboratory, Northern Regional Research Center, U.S Department of Agriculture, Peoria, Illinois 61604, and has been added to its permanent collection. It has been assigned the strain designation NRRL 18036 by said depository.

Culture LL-E19020 was isolated from a soil sample taken in a pasture near Lake Manyara, Tanzania, Africa. Culture LL-E19020 produces short spiral spore chains, 10-50 spores long, with occasional longer chains. These tend to coalesce to form dry blackish masses on such ISP media as oatmeal and inorganic salts-starch. The spores have smooth surfaces as assessed by electron microscopy. The strain contains the L isomer of diaminopimelic acid, and may thus be assigned to the genus Streptomyces.

In the ISP tests for utilization of carbohydrates, LL-E19020 shows growth on arabinose, fructose, inositol, mannitol, raffinose, rhamnose, sucrose and xylose. Cellulose is not utilized.

The reactions of LL-E19020 in the Gordon physiological series are compared in the following Table I with those of Streptomyces lydicus ISP 5461 which it most closely resembles morphologically and physiologically.

Because LL-E19020 differs from ISP 5461 in five characteristics (xanthine hydrolysis, decarboxylation of oxalate, acid from erythritol, rhamnose and beta-methyl-D-xyloside) it is designated as a subspecies of Streptomyces lydicus.

## TABLE I

### Gordon Test Reactions of LL-E19020

### and Streptomyces lydicus ISP 5461

| Reaction | LL-E19020 | ISP 5461 |
|---|---|---|
| **Degradation/Transformation of** | | |
| Casein | + | + |
| Xanthine | − | + |
| Hypoxanthine | + | + |
| Tyrosine | + | + |
| Adenine | + | + |
| **Production of** | | |
| Amylase | + | + |
| Gelatinase | + | + |
| Phosphatase | + | + |
| Nitrate Reductase | − | − |
| Urease | + | + |
| Esculinase | + | + |
| **Growth on/in** | | |
| 5% Sodium chloride | + | + |
| Salicylate | − | − |
| Lysozyme Broth | trace | trace |
| **Utilization of** | | |
| Acetate | + | + |
| Benzoate | − | − |
| Citrate | + | + |
| Lactate | + | + |

## TABLE I (continued)

| Reaction | LL-E19020 | ISP 5461 |
|---|---|---|
| Malate | + | + |
| Mucate | + | + |
| Oxalate | + | - |
| Propionate | + | + |
| Pyruvate | + | + |
| Succinate | + | + |
| Tartrate | - | - |
| **Growth at** | | |
| 10°C | + | + |
| 42°C | - | - |
| 50°C | - | - |
| **Acid from** | | |
| Adonitol | + | + |
| Arabinose | + | + |
| Cellobiose | + | + |
| Dextrin | + | + |
| Dulcitol | - | - |
| Erythritol | + | - |
| Fructose | + | + |
| Galactose | + | + |
| Glucose | + | + |
| Glycerol | + | + |
| Inositol | + | + |
| Lactose | + | + |
| Maltose | + | + |
| Mannitol | + | + |
| Mannose | + | + |
| Melibiose | + | + |
| α-Methyl-D-Glucoside | + | + |
| Raffinose | + | + |
| Rhamnose | + | - |
| Salicin | + | + |
| Sorbitol | + | + |
| Sucrose | + | + |
| Trehalose | + | + |
| Xylose | + | + |
| β-Methyl-D-Xyloside | + | - |

It is to be understood that for the production of these new antibacterial agents the present invention is not limited to this particular organism or to organisms fully answering the above characteristics which are given for illustrative purposes only. In fact, it is desired and intended to include the use of mutants produced from this organism by various means such as exposure to X-radiation, ultraviolet radiation, N'-methyl-N'-nitro-N-nitrosoguanidine, actinophages and the like.

6

The in vitro antibacterial activity of LL-E19020 alpha and beta was determined against a spectrum of gram-positive and gram-negative bacteria by a standard agar dilution method. Mueller-Hinton agar containing 5% sheep blood and two-fold decreasing concentrations of either LL-E19020 alpha or beta were poured into petri dishes. The agar surfaces were inoculated with 1 to $5\times10^4$ colony forming units of bacteria by means of the Steers replicating device. The lowest concentration of antibiotic that inhibited growth of a bacterial strain after 18 hours incubation was recorded as the minimal inhibitory concentration for that strain. The results are given in Table II.

TABLE II

In vitro Antibacterial Activity of LL-E19020 α and β

| Organism | | Minimal Inhibitory Concentration (mcg/ml) | |
|---|---|---|---|
| | | LL-E19020 α | LL-E19020 β |
| Staphylococcus aureus | ATCC 25923 | >256 | >256 |
| " | Smith | 128 | 128 |
| " | VGH-84-45 | >256 | >128 |
| " | CMC-83-127 | >256 | >128 |
| " | CMC-83-131 | >256 | >128 |
| " | CMC-83-132 | >256 | >128 |
| " | SSC-82-57 | >256 | >128 |
| " epidermidis | IO-83-58 | >256 | >128 |
| " saprophyticus | VHG-84-50 | >256 | >128 |
| Streptococcus sp. (β-hemolytic) | C203 | 0.5 | 0.5 |
| " | VGH-84-60 | 0.25 | 0.25 |
| " | VGH-84-61 | 1 | 0.5 |
| " | VGH-84-62 | 1 | 0.5 |
| " | VGH-84-63 | 0.12 | 0.12 |
| " | VGH-84-64 | 0.12 | 0.12 |
| " | SVI | 1 | 1 |
| Streptococcus pneumoniae | K-84-21 | 1 | 0.5 |
| " | VGH-84-56 | 1 | 0.5 |
| " | VGH-84-57 | 2 | 1 |
| " | VGH-84-58 | 0.25 | 0.25 |
| " | VGH-84-59 | 0.25 | 0.25 |

TABLE II (continued)

| Organism | | Minimal Inhibitory Concentration (mcg/ml) | |
|---|---|---|---|
| | | LL-E19020α | LL-E19020β |
| Enterococcus | VGH-84-65 | 256 | >128 |
| " | VGH-84-68 | >256 | >128 |
| " | IO-83-28 | >256 | >128 |
| " | IO-83-40 | >256 | >128 |
| Escherichia coli | CMC-83-72 | >256 | >128 |
| Klebsiella pneumoniae | 311 | >256 | >128 |
| Enterobacter cloacae | AD | >256 | >128 |
| Morganella morganii | VGH-84-37 | >256 | >128 |
| Serratia marcescens | VGH-84-71 | >256 | >128 |
| Pseudomonas aeruginosa | K-84-18 | >256 | >128 |
| Bacteroides fragilis | 12-4-4 | >128 | >128 |
| Clostridium difficile | NYC 77-1 | 4 | 1 |
| Clostridium perfringens | ATCC 17858 | 16 | 4 |
| Peptococcus magnus | ATCC 13124 | 0.12 | 0.5 |
| Peptococcus magnus | ATCC 29328 | 0.12 | 0.5 |

The in vivo antibacterial activity of antibiotics LL-E19020 alpha and beta was established by infecting female CD-1 mice from Charles River Laboratories, weighing 20±2 g each, intraperitoneally with either $1.7 \times 10^2$ CFU/0.5 ml of broth of Streptococcus pyogenes C203 or $6.5 \times 10^5$ CFU/0.5 ml of broth of Staphylococcus aureus Smith. The mice were treated subcutaneously, 30 minutes before infection with the indicated dose of the test compound in 0.5 ml of 0.2% aqueous agar. The results of this test appear in Table III.

8

## TABLE III

### In vivo Activity of LL-E19020α and β

| Single Subcutaneous Dose (mg/kg) | Survival Ratios 7 Days After Infection | | | |
| --- | --- | --- | --- | --- |
| | S. pyogenes C203 | | S. aureus Smith | |
| | LL-E19020α | LL-E19020β | LL-E19020α | LL-E19020β |
| 256 | NT | NT | 3/5 | 1/5 |
| 64 | 5/5 | 5/5 | 3/5 | 1/5 |
| 32 | 5/5 | 5/5 | NT | NT |
| 16 | 5/5 | 5/5 | 3/5 | 1/5 |
| 8 | 4/5 | 3/5 | NT | NT |
| 4 | 2/5 | 2/5 | 2/5 | 1/5 |
| Non-treated infected controls | 0/10 | 0/10 | 0/10 | 0/10 |

NT = not tested

Antibiotics LL-E19020 alpha and LL-E19020 beta derive their utility from their antibacterial activity. For example, these antibiotics may be used in the suppression of bacterial infections, as a topical antibacterial agent and as a general disinfectant for laboratories.

In therapeutic use, the compounds of this invention may be administered in the form of conventional pharmaceutical compositions appropriate for the intended use. Such composition may be formulated so as to be suitable for oral, parenteral, or topical administration. The active ingredient may be combined in admixture with a nontoxic pharmaceutically acceptable carrier, which carrier may take a wide variety of forms, depending on the form of preparation desired for administration, ie, oral, parenteral or topical.

In accordance with the invention, the antibiotics LL-E19020 alpha and LL-E19020 beta or salts thereof may be orally or parenterally administered to the animals. They may be administered in admixture with the animal's feed or as a top dressing therefore. They may also be proffered to said animals in the form of a bolus, pellet, tablet, pill, drench, oral gel or the like, or provided in the animal's drinking water.

When orally administered in or with the feed, generally about 0.1 to 300 grams of the antibiotic selected from LL-19020 alpha, LL-E19020 beta or a physiologically acceptable salt thereof per ton of feed is effective for enhancing the growth rate and improving the efficiency of feed utilization by the host animals.

Although the requirements of feed utilization of lactating ruminants such as dairy cows differ measurably from those of ruminants raised for meat production, surprisingly the concentration of antibiotic in feed, as described above for meat producing animals, is also effective for increasing lactation in lactating ruminants.

Ruminal VFA production is particularly important, since it relates directly to the normal maintenance of the animal, as well as to the quality and quantity of the milk produced by the animal. In the lactating ruminant, however, energy for lactation is the most limiting factor in milk production. Acetate is required for milk fat synthesis, while propionate is utilized to produce glucose, which in turn is required for lactose synthesis, and also has a minor role in milk fat production. Butyrate is more glycogenic than lipogenic, the lipogenic aspect being indirect since butyrate must first be degraded to acetate units before it can be utilized for long chain fatty acid synthesis, i.e., milk fat.

Accordingly, in order to increase milk production in lactating ruminants, it is necessary to increase propionate production, but not at a large expense of acetate and butyrate production. To this end it has now been established that oral administration of the above-said antibiotics to ruminants enhances the production of propionate in the rumen while simultaneously suppressing the production of acetate. As such, this treatment improves the propionate to acetate ratio in the animals rumen.

Since the antibiotics of the present invention are useful in the treatment of both monogastric and ruminant animals which may weigh only a few grams or as much as several thousand kilograms, the effective levels of antibiotic necessary for treating said animals will vary with the animals stage of development and from species to species. Effective levels for each animal species are therefore listed in Table IV below:

TABLE IV

Effective Antibiotic Levels For Different Animal Species

| Compound | Effective Feed Level g/ton | mg/hd/day | mg/kg body wt | Animals |
|---|---|---|---|---|
| LL-E19020 alpha or Salts Thereof | 0.1 - 300 | 0.1 mg - 25 grams | 0.001 - 50 | Cattle & Horses |
|  | 0.1 - 250 | 0.01 mg - 2.5 grams | 0.01 - 50 | Sheep, Goats & Swine |
|  | 0.1 - 200 | 0.001 mg - 100 mg | 0.01 - 50 | Chickens, Turkeys and Rabbits |
| LL-E19020 beta or Salts Thereof | 0.1 - 300 | 0.1 mg - 25 grams | 0.01 - 50 | Cattle & Horses |
|  | 0.1 - 250 | 0.01 mg - 2.5 grams | 0.01 - 50 | Sheep, Goats & Swine |
|  | 0.1 - 200 | 0.001 mg - 100 mg | 0.01 - 50 | Chickens, Turkeys and Rabbits |

Animal feed compositions which will provide the desired growth promotion and feed efficiency in the above-mentioned animals can be prepared by admixing the above said antibiotic or salt thereof, or an animal feed supplement containing said compound, with a sufficient quantity of an appropriate animal feed to provide the desired level of active compound in said feed.

Animal feed supplements can be prepared by admixing about 1.0% to 75% by weight of the antibiotic

EP 0 252 380 B1

or salt thereof, with about 99% to 25% by weight of carriers or diluents. Carriers or diluents suitable for use in the preparation of the feed supplements include the following: alfalfa meal, soybean meal, cottonseed oil meal, linseed oil meal, sodium chloride, corn meal, cane molasses, urea, bone meal, fish meal, corncob meal, calcium chloride, and other similar materials. Use of the carriers or diluents in feed supplements promote uniformity of distribution of the active ingredient in the finished feed into which the supplement is blended. It thus performs an important function by ensuring proper distribution of the active ingredient throughout the feed.

If the supplement is used as a top dressing for feed, it helps to ensure uniformity of distribution of the active material across the top of the dressed feed.

For parenteral administration, the antibiotic or antibiotic salt may be prepared in the form of a paste or pellet and administered as an implant, usually under the skin of the head or ear of the animal in which enhanced growth rate and/or improved efficiency of feed utilization is desired.

In practice, parenteral administration generally involves injection of a sufficient amount of the above said antibiotic or antibiotic salt to provide the animal with from about 0.0001 to 50 mg/kg of body weight of the active ingredient.

Paste formulations can be prepared by dispersing the antibiotic or antibiotic salt in a pharmaceutically acceptable oil, such as, for example, peanut oil, sesame oil and corn oil.

Pellets containing an effective level of the antibiotic LL-E19020 alpha or LL-E19020 beta can be prepared by admixing the above-said antibiotic with a diluent, such as carbowax, biodegradable polymers, carnauba wax, or the like. A lubricant, such as, magnesium stearate or calcium stearate may be added to improve the pelleting process if desired.

It is, of course, recognized that more than one pellet may be administered to an animal to achieve the desired dose level which will provide the increased growth rate and/or improve efficiency of feed utilization by said animal. Moreover, it has been found that additional implants may also be introduced periodically during the treatment period in order to maintain the proper drug release rate in the animal's body.

Administration of the above-identified antibiotics for control, treatment or prevention, of protozoan infections in meat-producing and companion animals, will generally be most practical in or with the feed or drinking water of the animals. However, said antibiotics can be given to the animals on an individual basis in the form of capsules, tablets, oral gels, or the like. They may also be administered parenterally, generally by subcutaneous injection, as a gel, paste, pellet, solution or the like, under the skin of the host animal.

In the practice of the present invention the antibiotics LL-E19020 alpha, LL-E19020 beta and the pharmaceutically and pharmacologically acceptable salts thereof, may be employed prophylactically, pharmaceutically or therapeutically for the control, prevention or inhibition of protozoal infections in poultry and ruminants. Generally about 0.1 ppm to 300 ppm, and preferably about 1 to 100 ppm of the antibiotic or antibiotic salt, in feed or drinking water, is effective for controlling protozoal infections, such as coccidiosis and Babesia in poultry, ruminants and companion animals.

Medicated animal feeds useful in the method of the present invention are usually prepared by thoroughly admixing about 0.00001% by weight to 0.03% by weight of the antibiotic LL-E19020 alpha or beta or salt thereof with a nutritionally balanced daily ration.

When using the compound of the invention for the prevention or control of protozoal infections, the active antiprotozoal agent is generally first prepared as an animal feed premix. The premix usually contains a relatively high percentage of the antiprotozoal agent and is generally blended with the animal's feed just prior to administration. If desired, the feed premix may also be applied as a top dressing for the animal's daily ration.

Feed premixes or concentrates, useful in the practice of the present invention, may be prepared by admixing about 1.0 to 15.0% by weight of the above-identified antibiotic, or a pharmaceutically and pharmacologically acceptable salt thereof, with about 99.0% to 85% by weight of a suitable carrier or diluent. Carriers suitable for use to make up the feed supplement compositions include the following: alfalfa meal, soybean meal, cottonseed oil meal, linseed oil meal, fish meal, sodium chloride, calcium carbonate, calcium sulfate, corn meal, cane molasses, urea, bone meal, corncob meal, rice hull meal, and the like. The carrier promotes an essentially uniform distribution of the active ingredient in the finished feed into which the supplement is blended. It thus performs an important function by ensuring proper distribution of the active ingredient throughout the feed.

In practice, usually one or more pounds of premix is added per ton of feed to obtain the desired level of antibiotic in the finished feed.

Since the compound of this invention and its pharmaceutically and pharmacologically acceptable salts are relatively insoluble in water, it is generally desirable, when administering the compound in the animal's drinking water, to dissolve the active compound in an organic solvent such as methanol, ethanol, acetone,

12

DMSO, oleic acid, linoleic acid, propylene glycol, or the like, and admix with the solution a small amount of surfactant and/or dispersing agent to assure solution and/or dispersion of the active ingredient in the animal's drinking water.

When administered to cattle, sheep, swine, poultry or companion animals on a mg/kg of body weight/day basis, generally about 0.0001 to 15 mg/kg of animal body weight per day, is effective for preventing or controlling protozoan infections in the above said animals. For prolonged treatment of animals, rates of from about 0.0001 mg/kg body weight/day to 5 mg/kg of animal body weight/day are usually employed.

Administration of the antibiotic or antibiotic salt will generally be most practical in or with the feed or in the drinking water. However, if desired, the active compounds or their pharmaceutically or pharmacologically acceptable salts may be administered to individual host animals in the form of a bolus, pill, tablet, drench, oral gel, capsule or the like. Advantageously, the active compounds can also be prepared in the form of a solution, pellet, paste, gel or the like and administered by injection usually subcutaneously under the skin of the animal generally in the vicinity of the head or ears of the host animal. While individual treatment of animals is less practical than group treatment via feed or drinking water, individual treatment is quite practical for use on a small scale, for example in the treatment of companion, domestic and circus animals and for zoological specimens.

When the antibiotics LL-E19020 alpha, LL-E19020 beta or salts thereof are used for prophylactic or therapeutic treatment of helminth or nematode infestations in meat producing animals such as cattle, sheep, goats, horses, swine or poultry or companion animals such as dogs, cats or the like, generally about 0.1 to 1000 ppm and preferably about 1 to 300 ppm of the antibiotic, administered in or with the diet or drinking water of the animal, is effective for preventing, controlling, or inhibiting helminth and nematode infestations in said animals.

For use in animal feeds, the antibiotics of the present invention are generally prepared as animal feed concentrates, premixes or supplements containing a relatively high percentage of the antibiotic admixed with edible carriers or diluents. These concentrates, premixes or supplements are then admixed with the feed just before administering the medicated feed.

Feed premixes, concentrates or supplements useful in the practice of the present invention, may be prepared by admixing about 1.0% to 25.0% by weight of the above-identified antibiotics, or pharmaceutically and pharmacologically acceptable salt thereof, with about 99.0% to 75% by weight of a suitable carrier or diluent as described above.

The carrier thus performs an important function by ensuring proper distribution of the active ingredient, i.e., about 0.1 ppm to 1000 ppm thereof, throughout the feed. This is equivalent to about 0.00001% to 0.1% by weight of the active ingredient in the finished feed. In practice, usually one or more pounds of premix is added per ton of feed to obtain the desired level of antibiotic in the finished feed.

Advantageously, where the treatment of a small number of the larger meat-producing animals is required to control helminths or nematodes therein, the antibiotics LL-E19020 alpha, LL-E19020 beta or pharmaceutically or pharmacologically acceptable salts thereof may be orally administered, on a daily basis to the host animal in the form of a medicated oral gel, a bolus, tablet, capsule, pill, drench or the like.

The antibiotic compounds of the invention have been found to be effective for controlling the free living nematode Caenorhabditis elegans and the sheep parasite Trichostrongylus colubriformis.

When administered to cattle, sheep, swine, poultry or companion animals on a mg/kg of body weight/day basis, generally about 0.1 to 100 mg/kg of animal body weight per day, is effective for preventing or controlling helminth or nematode infestations in the above said animals. For prolonged treatment of animals, rates of from about 0.0001 mg/kg body weight/day to 5 mg/kg of animal body weight/day are usually employed.

In practice, parenteral administration generally involves injection of a sufficient amount of the above said antibiotic or antibiotic salt to provide the animal with from about 0.1 to 100 mg/kg of body weight of the active ingredient.

General Fermentation Conditions

Cultivation of Streptomyces lydicus ssp. tanzanius NRRL 18036 may be carried out in a wide variety of liquid culture media. Media which are useful for the production of LL-E19020 alpha and LL-E19020 beta include an assimilable source of carbon, such as dextrin, sucrose, molasses, glycerol, etc; an assimilable source of nitrogen such as protein, protein hydrolysate, polypeptides, amino acids, corn steep liquor, etc; and inorganic anions and cations, such as potassium, sodium, ammonium, calcium, sulfate, carbonate, phosphate, chloride, etc. Trace elements such as boron, molybdenum, copper, etc., are supplied as

13

impurities of other constituents of the media. Aeration in tanks and bottles is supplied by forcing sterile air through or onto the surface of the fermenting medium. Further agitation in tanks is provided by a mechanical impeller. An antifoam agent such as silicon oil may be added as needed.

General Procedure for the Isolation of LL-E19020 alpha and beta

The LL-E19020 alpha and LL-E19020 beta are recovered from the fermentation broth by pH adjustment to 4.5-5.5, filtration through diatomaceous earth, extraction into a solvent such as ethyl acetate, concentration, dissolution in a solvent such as dichloromethane and purification by column chromatography on silica gel using successively, dichloromethane and methanol:dichloromethane (1:4), giving a crude product.

The crude product is then separated into the alpha and beta components and further purified by high performance liquid chromatography on a reverse-phase column using the system acetonitrile, 0.1M ammonium acetate buffer pH 4.3 (1:1).

The invention will be further described in conjunction with the following non-limiting examples.

Example 1

Inoculum Preparation

A typical medium used to grow the primary inoculum was prepared according to the following formula:
Dextrose .................... 1.0%
Dextrin ...................... 2.0%
Yeast extract ............ 0.5%
NZ Amine A®[1] .......... 0.5%
Calcium carbonate ... 0.1%
Water qs ................ 100.0%

This medium was adjusted to pH 7.0 and then sterilized. A 100 ml portion of this sterile medium in a 500 ml flask, was inoculated with mycelial scrapings from an agar slant of Streptomyces lydicus ssp. tanzanius NRRL 18036. The medium was then placed on a rotary shaker and incubated at 28°C for 48 hours. This primary inoculum was then used to inoculate 10 liters of the same sterile medium in a bottle. This medium was grown for 24 hours providing secondary inoculum. This secondary inoculum was then used to inoculate 250 liters of the same sterile medium in a tank. This medium was grown at 28°C for 48 hours with a sterile air flow of 200 liters per liter of mash per minute and agitation by an impeller driven at 220 rpm, providing tertiary inoculum.

Example 2

Fermentation

A fermentation medium of the following formulation was prepared:
Dextrin ...................... 3.0%
Molasses ................... 2.0%
Soy peptone .............. 0.75%
Yeast extract ............ 0.25%
Calcium carbonate .... 0.2%
Water qs ................... 100.0%

This medium was sterilized and 2700 liters was then inoculated with 300 liters of tertiary inoculum from Example 1. The fermentation was conducted at 28°C, with a sterile air flow of 0.55 liters of air per liter of mash per minute and agitation by an impeller driven at 100 rpm for 113 hours, at which time the mash was harvested.

Example 3

Isolation and Purification of LL-E19020$\alpha$ and $\beta$

[1][A pancreatic digest of casein, registered trademark of Sheffield Chemical, Norwich, NY]

The harvest mash from two fermentations conducted as described in Example 2 were combined, making a total of 6000 liters, adjusted to pH 5 with hydrochloric acid and filtered through diatomaceous earth. The filtrate was extracted with ethyl acetate and the extract concentrated to a syrup.

This syrup was dissolved in dichloromethane and applied to 1000 g of silica (60-200 mesh) on a sintered glass funnel. The silica column was first eluted with dichloromethane, collecting four 2 liter fractions and then with methanol:dichloromethane (1:4) collecting a 4 liter fraction. This 4 liter fraction was evaporated to dryness, giving 120 g of residue. The residue was redissolved in 4 liters of dichloromethane and applied to 500 g of silica on a sintered glass funnel. The silica was eluted with methanol:dichloromethane (1:4) collecting 2 liter fractions. Fractions 1 and 2 were combined and evaporated, giving 99 g of crude LL-E19020$\alpha$ and $\beta$.

This crude product was dissolved in methanol and applied to a 12 liter reverse-phase column (C18 bonded phase 40 micron). The column was eluted with acetonitrile, 0.1M ammonium acetate buffer pH 4.3 (1:1) at a rate of 1.0 liters per minute. Thirteen 24 liter fractions were collected. Fraction 7 contained LL-E19020$\alpha$ and fractions 11-13 contained LL-E19020$\beta$.

The antibiotics were extracted from the mobile phase using dichloromethane followed by evaporation and freeze drying from t-butanol, giving 10 g of LL-E19020$\alpha$ and 14 g of LL-E19020$\beta$, both as white solids.

Example 4

PASTEURELLA DISK TEST

Sterile paper disks (1/4" in diameter) are soaked in a 2.5 mg/ml solution of test compound and dried in a 37°C incubator overnight. Standard antibiotic control disks are prepared for testing along with the test compound disks. The dried disks are stored at 2-4°C until used. Two test organisms, Pasteurella multocida 31081B and Pasteurella haemolytica 30660, are cultured in brain heart infusion broth for 5 hours at 37°C. A 1:10 dilution of each culture is made in Mueller-Hinton broth. Two hundred milliliters of Mueller-Hinton agar are seeded with 1 ml of the diluted culture and aseptically poured into 9 inch x 9 inch bioassay plates manufactured by Nunc. Use of the 9" x 9" plates permits the testing of 36 disks per plate. Appropriate disks are applied to the seeded agar plates and incubated for 18-20 hours at 37°C. Zones of inhibition are recorded.

T. HYODYSENTERIAE DISK TEST

Sterile paper disks (1/4" in diameter) are soaked in a 2.5 mg/ml solution of a test compound and dried in a 37°C incubator overnight. Standard antibiotic control disks are prepared for testing along with the test compound disks. The dried disks are stored at 2-4°C until used. Two T. hyo. strains, B78 (ATCC 27164) and B204 (ATCC 31212), are cultured for 24 hours at 38°C in Hungate culture tubes containing 5 ml brain heart infusion broth supplemented with 2% fetal calf serum (prepared anaerobically). Two hundred milliliters of trypticase soy agar, containing 5% defibrinated bovine blood, are seeded with 1 ml of culture and aseptically poured into 9" x 9" bioassay plates manufactured by Nunc. Use of the 9" x 9" plates permit the testing of 36 disks per plate. Appropriate disks are applied to the agar plates which are then incubated for 24-48 hours at 38°C in an anaerobic chamber containing 80% nitrogen, 10% carbon dioxide, and 10% hydrogen until hemolysis is complete. Zones of inhibited hemolysis are recorded.

MINIMUM INHIBITORY CONCENTRATION PROCEDURE BY AGAR DILUTION

1. Serial two-flow dilutions of drug are prepared in Mueller-Hinton broth in a range of 2560 $\mu$g/ml - 0.15 $\mu$g/ml plus a solvent control.

2. Two milliliters of drug dilution (10X) are added to sterile screwcap bottles to which 18 ml of Mueller-Hinton agar containing 5.6% defibrinated sheep blood is added. Final drug concentration ranges 256 $\mu$g/ml - 0.015$\mu$g/ml in agar containing 5% sheep blood.

3. A few isolated colonies of each test organism are inoculated into 5 ml trypticase soy broth or brain heart infusion broth. The cultures are shaken at 35°C for 5 hours.

4. Each culture is diluted 1:50 ($10^{-1.7}$) in Mueller-Hinton broth and applied to agar plates using a Steers replicator. Control plates should be seeded last to ensure that viable organisms were present throughout the procedure. Inoculated agar plates are allowed to stand undisturbed until the inoculum spots are completely absorbed.

5. The plates are inverted and incubated at 35°C for 18 hours without $CO_2$.

6. The minimum inhibitory concentration (MIC) is taken as the lowest concentration of antimicrobial agent at which complete inhibition occurs. A very fine, barely visible haze or a single colony is disregarded.

MIC TEST ORGANISMS

Staphylococcus aureus ATCC 25923
Staphylococcus aureus 52 "Smith strain"
Staphylococcus aureus 14 ATCC 6538P
Staphylococcus aureus 335 Mastitis isolate
Staphylococcus aureus 336 Mastitis isolate
Staphylococcus aureus 344 Mastitis isolate
Staphylococcus aureus Penicillin resistant
Streptococcus pyogenes ATCC 19615
Streptococcus pyogenes 41
Streptococcus agalactiae 341
Streptococcus agalactiae 342
Streptococcus agalactiae 343
Streptococcus dysgalactiae 340
Streptococcus faecalis 42 Dr. Juke's #8043
Streptococcus uberis Cornell Mastitis Center
Escherichia coli ATCC 25922
Escherichia coli 81
Escherichia coli 80-654 Tetracycline resistant
Pasteurella multocida 31081B (in vitro disk test strain)
Pasteurella multocida 80-3548 (in vivo mouse model strain)
Pasteurella multocida 31451
Pasteurella multocida 32301
Pasteurella multocida 30170B
Pasteurella multocida 80-5945
Pasteurella haemolytica 30660 (in vitro disk test strain)
Pasteurella haemolytica L-101 National Animal Disease Center
Pasteurella haemolytica 80-6744
Salmonella choleraesuis var. Kunzendorf I-3
Salmonella choleraesuis var. Kunzendorf 4
Bordetella bronchiseptica "B" strain
Bordetella bronchiseptica 11266
Bordetella bronchiseptica 31068B
Bordetella bronchiseptica 11948A

MINIMUM INHIBITORY CONCENTRATION ASSAY FOR Mycloplasma gallisepticum

1. Serial two-fold dilutions of drug stock solutions are prepared in mycoplasma broth in a concentration of 2560 $\mu$g/ml - 0.015 $\mu$g/ml plus a solvent control. These concentrations are 10X the final test concentration.

2. A frozen (-80°C) stock culture of Mycoplasma gallisepticum "R" strain is thawed and a 0.5 ml aliquot is inoculated into 5 ml of mycoplasma culture broth. At the same time, 0.1 ml is plated on to a mycoplasma agar plate as a purity check. Both cultures are incubated at 37°C. Growth in broth is indicated by a color change from red to yellow. Growth on agar is observed with the aid of a stereoscope.

3. The MIC assay is carried out in 96 well microtiter plates. To each test well, 25 $\mu$l of 10X drug solution is aliquoted. Appropriate solvent controls are also included.

4. The mycoplasma inoculum is prepared by transferring a positive broth culture to fresh medium using the ratio of 0.2 ml culture:5.0 ml medium. Large amounts of inoculum are prepared as needed using the formula above.

5. A 225 $\mu$l aliquot of previously inoculated mycoplasma broth is added to each test well and mixed. A plastic sealer tape is applied and a small hole is placed over the center of each test well using sterile 25 guage needles. To avoid well cross-contamination, needles are changed for each drug. Further, tape puncturing proceeds from lowest to highest concentration of drug. Final test concentration ranges from

16

256 µg/ml-0.0015 µg/ml with a total volume of 250 µl. Wells containing 250 µl of inoculated medium only and uninoculated medium are added as further controls.

6. The assay plate is incubated at 37°C until a broth color change from red to yellow first occurs uniformly throughout the test plate. The MIC value is recorded as the concentration at which the broth color (red) remains unchanged.

TABLE V

ANTIBACTERIAL DATA FOR E19020 ALPHA & E19020 BETA

**PRIMARY IN VITRO DISK DIFFUSION TEST**

ZONE SIZE (mm)

| | E19020 ALPHA | E19020 BETA | CTC | TIAMULIN |
|---|---|---|---|---|
| Pasteurella multocida 31081B | 15 | 13 | 25 | N/T |
| Pasteurella haemolytica 30660 | 13 | 13 | 11 | N/T |
| Treponema hyodysenteriae B78 (ATCC 27164) | 0 | N/T | 45 | 67 |
| Treponema hyodysenteriae B204 (ATCC 31212) | 15 | 11 | 37 | 70 |

N/T = Not Tested

TABLE VI

| MINIMUM INHIBITORY CONCENTRATIONS BY AGAR DILUTION | | |
|---|---|---|
| | MIC ($\mu$g/ml) | |
| | E19020 ALPHA | E19020 BETA |
| Staphylococcus aureus (7) | $\geq$ 256 | 128 - $\geq$ 256 |
| Streptococcus pyogenes (2) | 2 | 2 - 4 |
| Streptococcus agalactiae (4) | 2 - 8 | 2 - 4 |
| Streptococcus faecalis (1) | 8 | 8 |
| Streptococcus uberis (1) | 8 | 4 |
| Escherichia coli (2) | $\geq$ 256 | $\geq$ 256 |
| Salmonella choleraesuis (2) | > 256 | > 256 |
| Bordetella bronchiseptica (4) | > 256 | > 256 |
| Pasteurella multocida (6) | 8 - 16 | 8 - 16 |
| Pasteurella haemolytica (3) | 16 | 16 |
| NOTE: Numbers in parentheses indicate the number of strains tested. | | |

TABLE VII

MINIMUM INHIBITORY CONCENTRATION BY MICRODILUTION

MIC (μg/ml)

| | E19020 ALPHA | E19020 BETA | CARBADOX | TIAMULIN | CTC | TYLOSIN |
|---|---|---|---|---|---|---|
| Treponema hyodysenteriae B78 (ATCC 27164) | 1 | 1 | 0.5 | 0.015 | N/T | N/T |
| Treponema hyodysenteriae B204 (ATCC 31212) | 1 | 0.5 | 0.25 | 0.015 | N/T | N/T |
| Mycoplasma gallisepticum "R" | 0.125 | 0.125 | N/T | N/T | 0.5 | 0.03 |

N/T = Not Tested

EXAMPLE 5

Evaluation of test compounds for increasing the growth of chickens and improving the efficiency of feed utilization thereby.

In this test one day old Peterson X Arbor Acres chicks are sorted into equal weight groups of 5 males and 5 females per cage. Cages are randomized to treatment groups with six replicates per treatment. Each compound is tested at 50 and 100 ppm in the diet and evaluated against chicks receiving a non-medicated diet.

The chicks are weighed at the start of the test and at 1 week intervals thereafter to the conclusion of said test. The chicks are given free access to feed and water during the entire test period. The feed is weighed when provided to the chicks and excess feed collected and weighed when the cages are cleaned.

The poultry diet employed in the test is as follows:

Vitamin-amino acid premix 0.5%
Trace minerals 0.1%
Sodium chloride 0.3%
Dicalcium phosphate 1.2%
Ground limestone 0.5%
Stabilized fat 4.0%
Dehydrated alfalfa, 17% protein 2.0%
Corn gluten meal, 41% protein 5.0%
Menhaden fish meal, 60% protein 5.0%
Soybean oil meal, 44% protein 30.0%
Ground yellow corn, fine to 100.0%

The vitamin-amino acid premix in the above feed composition is prepared from the following formulation. The expressions of quantity relate to units per kilogram of the finished feed compositions.

Butylated hydroxy toluene 125.0 mg
d1-Methionine 500.0 mg
Vitamin A 3300.0 I.U.
Vitamin $D_3$ 1100.0 I.C.U.
Riboflavin 4.4 mg
Vitamin E 2.2 I.U.
Niacin 27.5 mg
Panthothenic acid 8.8 mg
Choline chloride 500.0 mg
Folic acid 1.43 mg
Menadione sodium bisulfate 1.1 mg
Vitamin $B_{12}$ 11.0 mcg
Ground yellow corn, fine to 5.0 mg

Data obtained are reported in Table VIII below where it can be seen that antibiotics LL-E19020 alpha and LL-E19020 beta both improved the weight gains of chicks and increased the efficiency of feed utilization thereby over unmedicated controls.

20

TABLE VIII

Evaluation of Test Compounds For Increasing The Growth Rate of Poultry And Improving The Efficiency of Feed Utilization Thereby.

| Treatment | ppm | Weight Gains % Improvement over Controls | | | Feed/Gain | | |
|---|---|---|---|---|---|---|---|
| | | 0-1 Wk | 0-2 Wk | 0-3 Wk | 0-1 Wk | 0-2 Wk | 0-3 Wk |
| Control | - | - | - | - | 1.21 | 1.33 | 1.43 |
| Antibiotic (E 19020 alpha) | 50 | 12.5** | 4.1* | 4.2* | 1.11* | 1.30 | 1.38 |
| | 100 | 8.9* | 6.3** | 6.1** | 1.12* | 1.27 | 1.39 |
| Antibiotic (E 19020 beta) | 50 | 12.7** | 4.5* | 5.3* | 1.11* | 1.31 | 1.40 |
| | 100 | 10.7* | 5.1* | 5.9** | 1.13* | 1.29 | 1.38 |

\* - p=.05
\*\* - p=.01

EXAMPLE 6

Evaluation of test compounds for increasing the weight gain of poultry and improving the efficiency of feed utilization thereby.

Five male and five female one day old Peterson X Arbor Acres chicks are allotted to starter battery cages by weight. Cages are randomized to treatment groups with seven replicates per treatment. Each compound is tested at 12.5, 25, 50, 100, and 200 ppm in a diet. The positive standard is penicillin at 200 ppm. Diets are prepared weekly. This study has unusually high mortality (6.2%). Mortality is usually less than 3%. The high mortality is apparently due to toe clipping the males for identification since male deaths outnumbered female deaths by 3:1 ratio. In addition male weight gain improvements were lower than female weight gain improvements, which is not the case in the first study. The data is summarized in Tables IX and IX A.

The diet employed in this test is the same as described in Example 5 above.

TABLE IX

| Weight Gain And Feed Efficiency Of Broiler Chicks Treated With Antibiotics LL-E19020 Alpha And LL-E19020 Beta | | | | | |
|---|---|---|---|---|---|
| TREATMENT | PPM | Wg Gains And Feed Efficiency % Improvement Over Controls | | | |
| | | FEMALES | MALES | COMBINED | FEED/GAIN |
| Control | 0 | - | - | - | 1.41 |
| Penicillin | 200 | 4.2 | 2.4 | 3.5 | 1.35** |
| E19020 alpha | 12.5 | 7.8* | 0 | 3.8 | 1.36* |
| E19020 alpha | 25 | 4.8 | 5.9 | 5.5 | 1.34** |
| E19020 alpha | 50 | 10.5** | 3.6 | 6.6* | 1.34** |
| E19020 alpha | 100 | 3.2 | 2.9 | 2.9 | 1.34** |
| E19020 alpha | 200 | 7.1 | 4.5 | 5.7 | 1.32** |
| E19020 beta | 12.5 | 6.0 | 0 | 2.6 | 1.33** |
| E19020 beta | 25 | 5.1 | 2.4 | 3.4 | 1.38* |
| E19020 beta | 50 | 4.6 | 0 | 2.2 | 1.38* |
| E19020 beta | 100 | 7.2 | 1.7 | 4.5 | 1.34** |
| E19020 beta | 200 | 1.4 | 4.2 | 2.7 | 1.35** |

\* p = .05
\*\* p = .01

Following the above procedure but altering the rates of administration, and extending the test period to 7 weeks, feed efficiency and weight gains of chicks was again determined and data obtained are reported in Table IIA below.

TABLE IX A

WEIGHT GAINS IN BROILER CHICKS TREATED WITH E19020 ALPHA

| | | 0-3 WEEKS | | 0-7 WEEKS | |
|---|---|---|---|---|---|
| | PPM | WEIGHT GAIN IN GRAMS | % IMPROVEMENT OVER CONTROLS | WEIGHT GAIN IN GRAMS | % IMPROVEMENT OVER CONTROLS |
| Control | 0 | 608 | - | 2145 | - |
| Penicillin | 200 | 638* | 4.9 | 2217 | 3.4 |
| E19020 alpha | 6.25 | 635* | 4.4 | 2183 | 1.8 |
| | 12.5 | 647* | 6.3 | 2198 | 2.5 |
| | 25 | 659** | 8.4 | 2267* | 5.7 |
| | 50 | 642* | 5.6 | 2206 | 2.8 |
| | 100 | 652** | 7.3 | 2204 | 2.8 |

EP 0 252 380 B1

## TABLE IX A (Continued)

### FEED EFFICIENCY IN BROILER CHICKS TREATED WITH E19020 ALPHA

| | | 0-3 WEEKS | | 0-7 WEEKS | |
|---|---|---|---|---|---|
| | PPM | FEED/GAIN | % IMPROVEMENT OVER CONTROLS | FEED/GAIN | % IMPROVEMENT OVER CONTROLS |
| Control | 0 | 1.43 | - | 1.99 | - |
| Penicillin | 200 | 1.38** | 3.5 | 1.87** | 6.0 |
| E19020 alpha | 6.25 | 1.38** | 3.5 | 1.89** | 5.0 |
| | 12.5 | 1.38** | 3.5 | 1.91** | 4.0 |
| | 25 | 1.38** | 3.5 | 1.89** | 5.0 |
| | 50 | 1.39* | 2.8 | 1.91** | 4.0 |
| | 100 | 1.35** | 5.6 | 1.91** | 4.0 |

\* $p < .05$
\*\* $p < .01$

E19020 alpha lot #6951C-86A

## EXAMPLE 7

Determination of Propionate Enhancement Activity By Oral Administration Of Test Compounds.

In these tests strained rumen fluid (2.5 ml) obtained from a fistulated steer is incubated anaerobically at 39°C with 2.5 ml of McDougall's artificial saliva buffer containing 12.5 mg/ml of substrate (60.5% corn

24

starch, 25.9% essential amino acid mixture and 13.6% alpha-cellulose) and appropriate concentrations of drug for 20-24 hr in a shaking water bath using sealed vials with gas release valves. The drugs are dissolved or suspended (sonified if necessary) in a 0.1% Tween 20-ethanol-water vehicle and 25-50 ul is added to the incubation. The reaction is terminated with 6N HCl and centrifuged at 20,000 x g. Alliquots of the superinatant are analyzed for volatile fatty acid (VFA) content by gas-liquid chromatography.

Compounds causing an enhancement of propionate production determined by a decrease in the acetate/propionate ratio determined by a decrease in the acetate/propionate ratio in this test have generally been found to increase feed efficiency in ruminants. The data is summarized in Tables X and XI.

TABLE X

Propionate Enhancement Activity Of Test Compounds During In Vitro Rumen Fermentation.

| TREATMENT[a] (PPM) | TOTAL VFA (mM) | (S.D.) | A:P | % DECREASE FROM CONTROL A:P |
|---|---|---|---|---|
| Control[b] | 159.4 | 4.2 | 2.65 | --- |
| Antibiotic LL-E19020 alpha | | | | |
| 0.008 | 161.2 | 0.2 | 2.66 | 0.0 |
| 0.031 | 158.4 | 5.4 | 2.43 | 8.4 |
| 0.125 | 159.2 | 1.8 | 2.14 | 19.1 |
| 0.500 | 146.9 | 8.1 | 2.27 | 14.1 |
| 2.000 | 150.5 | 2.0 | 2.21 | 16.5 |
| Antibiotic LL-E19020 beta | | | | |
| 0.008 | 161.2 | 0.0 | 2.63 | 0.6 |
| 0.031 | 161.1 | 0.6 | 2.44 | 7.8 |
| 0.125 | 161.6 | 2.0 | 2.19 | 17.4 |
| 0.500 | 156.7 | 3.2 | 2.15 | 18.8 |
| 2.000 | 153.8 | 5.6 | 2.18 | 17.6 |

TABLE X (Cont.)

Propionate Enhancement Activity Of Test Compounds
During In Vitro Rumen Fermentation.

| TREATMENT[a] | TOTAL VFA | | | % DECREASE FROM |
|---|---|---|---|---|
| (PPM) | (mM) | (S.D.) | A:P | CONTROL A:P |
| AVOPARCIN | | | | |
| 1.0 | 159.4 | 1.8 | 2.38 | 10.2 |
| 2.0 | 160.2 | 0.9 | 2.23 | 15.9 |
| 4.0 | 158.9 | 1.6 | 2.12 | 19.8 |
| 8.0 | 158.3 | 3.2 | 2.08 | 21.3 |
| CONTROL(ACID) | 87.1 | 0.4 | 3.73 | --- |

[a]n=2 unless indicated differently.
[b]n=6.

EP 0 252 380 B1

TABLE XI

Propionate Enhancement Activity Of Test Compounds
During In Vitro Rumen Fermentation.

| TREATMENT[a] (PPM) | TOTAL VFA (mM) | (S.D.) | A:P | % DECREASE FROM CONTROL A:P |
|---|---|---|---|---|
| Control[b] | 125.3 | 3.6 | 2.19 | --- |
| Antibiotic LL-E19020 alpha | | | | |
| 0.015 | 125.1 | 1.4 | 2.05 | 6.4 |
| 0.031 | 126.5 | 1.5 | 1.96 | 10.7 |
| 0.062 | 126.3 | 2.5 | 1.90 | 13.0 |
| 0.125 | 121.9 | 1.0 | 1.92 | 12.1 |
| 0.250 | 121.1 | 1.2 | 1.85 | 15.5 |
| Antibiotic LL-E19020 beta | | | | |
| 0.015 | 127.2 | 2.4 | 2.03 | 7.2 |
| 0.031 | 125.7 | 1.1 | 1.98 | 9.5 |
| 0.062 | 123.7 | 0.4 | 1.96 | 10.3 |
| 0.125 | 124.3 | 2.1 | 1.92 | 12.2 |
| 0.250 | 122.6 | 1.5 | 1.93 | 11.9 |

EP 0 252 380 B1

TABLE XI (Cont.)

Propionate Enhancement Activity Of Test Compounds During In Vitro Rumen Fermentation.

| TREATMENT[a] (PPM) | TOTAL VFA (mM) | (S.D.) | A:P | % DECREASE FROM CONTROL A:P |
|---|---|---|---|---|
| AVOPARCIN | | | | |
| 1.0 | 126.9 | 1.7 | 2.02 | 7.6 |
| 2.0 | 125.3 | 3.1 | 1.98 | 9.4 |
| 4.0 | 121.3 | 2.0 | 1.90 | 13.1 |
| 8.0 | 119.4 | 0.5 | 1.88 | 14.1 |
| CONTROL(ACID) | 48.7 | 1.1 | 4.41 | --- |

[a]n=3 unless indicated differently.
[b]n=9.

EXAMPLE 8

Evaluation of test compounds as anticoccidial agents - Eimeria tenella

Anticoccidial Cell Culture Screen (E. tenella)

Monolayers are initiated from primary kidney cells obtained from 7 day old chickens. Generally, 50,000 cells are administered in 1 mL volumes of culture medium into 24 well cluster plates. The medium of preference is M199 buffered with 0.125% $NaHCO_3$. 5% fetal bovine serum is added to complete the medium.

Monolayers are permitted to grow to 50% confluency prior to inoculation and drug medication. This usually requires 48 hours in a humidified incubator held at 41 °C in an atmosphere of 5% $CO_2$-95% air.

28

Inocula containing 60,000 E. tenella sporozoites per 1 mL (2 mL total volume) is utilized to infect adequately confluent monolayers. Initial medium is aspirated off prior to administering 1.9 mL of sporozoite containing maintenance medium.

Medication is administered at predetermined concentrations in 0.1 mL volumes immediately after the introduction of sporozoites. As a rule synthetic compounds are tested at 1 ppm and crude fermentation products at a dilution of 1:200. The former are dissolved in DMSO and the latter in phosphate buffered saline. Penicillin and streptomycin are added to all medium to control possible contamination.

Treatments are observed by inverted phase contrast microscopy at 96-120 hours post-inoculation for cytotoxicity and anticoccidial activity. No staining of monolayers is required. Agents which prevent or markedly reduce the development of second generation asexual stages of the parasite life cycle are considered active. Medications which prevent an anticoccidial reading due to gross cytotoxicity are diluted 2 fold until end-points are achieved. Actives are generally diluted to inactivity to determine relative potency.

Monensin and robenidine are included in all experiments as positive standards. Data obtained are reported in Table XII below.

Table XII

| Evaluation of Test Compounds Using In Vitro Anticoccidial Screen - Eimeria tenella | | | | | | |
|---|---|---|---|---|---|---|
| Compound | Results in ppm of Culture Medium | | | | | |
| | 5 | 2.5 | 1.25 | 0.6 | 0.3 | 0.15 |
| LL-E19020 alpha | T | T | A | A | a | O |
| LL-E19020 beta | T | T | T | T | A | A |

Performance Rating T = Toxic A = Active a = Marginal Activity O = Inactive

EXAMPLE 9

Evaluation of test compounds as anticoccidial agents - Eimeria mitis

Anticoccidial Embryonated Egg Assay - E.mitis

Chick embryros which are 10 days of age are candled for viability and placed into groups of 5 eggs each. A small hole is punched into the chorioallantoic cavity as an avenue for chemical agents. Synthetic compounds are screened at 1 mg per embryo and crude natural products at 0.2 mL per embryo. Penicillin/streptomycin is utilized to control contamination. After administration of drugs via sterile tuberculin syringe, the pin hole is sealed with collodion. The embryos are returned to a humidified incubator set at 103°F and held for 24 hours. All embryos are then recandled to remove any dead due to toxicity of drug treatment. All surviving embryos are then inoculated through the original pin hole with 80,000 E.mitis sporozoites in a volume of 0.1 mL. Embryos are resealed and returned to the incubator. After 6 days all embryos are again candled and the dead removed and recorded. The chorioallantoic (CAM) membranes from each treatment are pooled and homogenized in 50-70 mL of tap water. Oocyst counts are then performed with a hemocytometer. A treatment is considered active if there is a 80% or greater reduction of oocysts compared to the numbers detected in the 4 replicates of nonmedicated inoculated controls. Two or more embryos must survive for a valid anticoccidial reading. In groups where one or no eggs remain after 6 days two fold dilutions are performed until an anticoccidial reading can be realized. Robenidine is used as a positive drug at 0.1 mg per embryo.

The E.mitis utilized has been made adaptable to this assay through repeated passages in embryos. (P.L. Long).

Data obtained are reported in Table XIII below.

Table XIII

| Evaluation of Test Compounds as Anticoccidial Agents - Eimeria mitis | | | |
|---|---|---|---|
| Compound | Milligrams of Drug Per Embryo | | |
| | 1 | 0.5 | 0.25 |
| LL-E19020 alpha | A | A | O |
| LL-E19020 beta | O | | |
| Performance Rating | | | |
| T = Toxic | | | |
| A = Active | | | |
| a = Marginal Activity | | | |
| O = Inactive | | | |

EXAMPLE 10

Evaluation of test compounds for controlling Babesia bovis and Babesia bigemina

For these evaluations laboratory cultures of Babesia bovis and Babesia bigemina are continuously maintained. A normal donor animal is maintained as a source of normal RBC's and serum.

Babesia cultures are maintained as follows:

1. The percent of Babesia growing in each flask is determined. If the percent is under 10% the cultures are fed.

2. When the percent of Babesia in a flask is between 10-20% and the cells and Babesia look healthy the culture should be split or used for the drug screening.

A. Media for Babesia cultures

30 mL 199(Earls) Media

20 mL serum

1 mL TES

Check pH (7.00) and adjust if necessary Filter sterilize

B. Feeding of Babesia cultures

Always put exactly the same amount of media in a culture as you remove.

C. Splitting Babesia cultures

Remove media from flask but do not remove any of the RBC. Add the exact amount removed of media + serum + tes. Mix gently.

Add 10 mL of the above gently mixed Babesia culture to 30 mL of 199 media/serum/tes and 3 mL RBC. Split Babesia culture 1:4.

Depending on the size of the flask used the amount of split Babesia culture necessary to maintain a viable culture.

200 mL = 45 mL split Babesia culture

30 mL = 15 mL split Babesia culture

Preparation of Babesia Cultures for Drug Screening

1. Make up 50 mLs Media 199 + serum + tes (pH and filter).

2. Make slides of infected cultures and determine the % of infected cells.

3. Make up a 5% suspension and a 6.7% suspension or normal bovine RBS's in Media 199 + serum + tes.

4. Dilute infected cells in the 6.7% suspension or normal RBC to obtain a final suspension containing 4% infected cells.

Preparation of microtiter plates.

1. Label plate as follows:

a. Row A  =  200 $\mu$l 5% suspension normal RBC

b. Row B  =  150 $\mu$l 4% suspension infected cells  +  50 $\mu$l media.

c. Row C  =  150 $\mu$l 4% suspension infected cells  +  50 $\mu$l drug conc.

d. Row D  =  150 $\mu$l 4% suspension infected cells  +  50 $\mu$l drug conc.

e. Row E  =  150 $\mu$l 4% suspension infected cells  +  50 $\mu$l drug conc.

f. Row F  =  150 $\mu$l 4% suspension infected cells  +  50 $\mu$l drug conc.

g. Row G  =  150 $\mu$l 4% suspension infected cells  +  50 $\mu$l drug conc.

h. Row H  =  150 $\mu$l 4% suspension infected cells  +  50 $\mu$l drug conc.

2. After plate is complete place in the incubator over night (18 hrs)

3. Add 25 $\mu$l of $^3$H hypoxanthine to each well the next morning.

4. Place the plate in the freezer at the end of the day.

5. Harvest the plate, after incubating for 2 hrs, and prepare for counting in the scintillation counter.


Preparation of Drug

1. Weight out 10 mg of drug - place in sterile 15 mL tube. Add 10 mL of media 199  +  serum  +  tes to make a concentration of 1 mg/mL or 1000 $\mu$g/mL.

Note:     if not soluble use 10 mL of 70%ETOH  +  30% $H_2O$

2. Filter.

| | | Drug Conc. | Dil factor | End Conc. in culture |
|---|---|---|---|---|
| | a. | 1000 $\mu$g/mL | none | 150 $\mu$g/mL |
| | b. | 500 $\mu$g/mL | 1:2 of a | 125 $\mu$g/mL |
| | c. | 200 $\mu$g/mL | 1:5 of a | 50 $\mu$g/mL |
| | d. | 100 $\mu$g/mL | 1:10 of a | 25 $\mu$g/mL |
| | e. | 50 $\mu$g/mL | 1:10 of b | 12.5 $\mu$g/mL |
| | f. | 20 $\mu$g/mL | 1:10 of c | 5 $\mu$g/mL |
| then dilute the 1000 glmL 1:50  =  20 $\mu$g/mL | | | | |
| | a. | 20a $\mu$g/mL | none | 5 $\mu$g/mL |
| | b. | 10 $\mu$g/mL | 2:2 of a | 2.5 $\mu$g/mL |
| | c. | 5 $\mu$g/mL | 1:2 of b | 1.25 $\mu$g/mL |
| | d. | 2.5 $\mu$g/mL | 2:2 of c | 0.625 $\mu$g/mL |
| | e. | 1.25 $\mu$g/mL | 2:2 of d | 0.31 $\mu$g/mL |
| | f. | 0.625 $\mu$g/mL | 2:2 of e | 0.155 $\mu$g/mL |

Anti-Babesia bovis Activity

Procedure: Babesia bovis is maintained in in vitro culture. The cultures were diluted to a parasitemia of 4% and cultured with various dilutions of the drugs. After incubation for 18 hours 3H hypoxanthine was added and the cultures incubated for an additional 18 hours. The plates were harvested and the samples counted. Growth inhibition results in a decreased incorporation of 3H hypoxanthine. The drugs were evaluated on duplicate plates and at 2 different time intervals. With this procedure, the 50% inhibitory concentration of LL-E19020 alpha was found to be 0.625 $\mu$g/mL.

Anti-Babesia bigemina Activity

Procedure: Babesia bigemina is maintained in in vitro culture. The cultures were diluted to a parasitemia of 4% and cultured with various dilutions of the drugs. After incubation for 18 hours 3H hypoxanthine was added and the cultures incubated for an additional 18 hours. The plates were harvested and the samples counted. Growth inhibition results in a decreased incorporation of 3H hypoxanthine can be seen in Fig. XIV. The drugs were evaluated on duplicate plates and at 2 different time intervals.

EXAMPLE 11

Evaluation of Test Compounds For Controlling The Free Living Nematode Caenorhabditis elegans

Culture Maintenance: Cultures of C. elegans (Bristol strain from J. Lewis) are maintained on E. coli lawns on NG Agar Plates at 20 C. New cultures are established weekly.

Nematodes for testing are washed from 4-5 day old cultures using Fresh Ascaris ringers Solution (FARS). The worms are further washed with FARS, containing gentamycin, to reduce bacterial contamination and centrifuged to separate worms from wash solution. This procedure is repeated three times. The washed worms are than added to C. briggsae Maintenance Medium (CbMM), from GIBCO[a]to which is added gentamycin (600 units/ml) and mycostatin (0.5 mg.ml).

Compounds are dissolved in acetone and made up to volume with equal parts of water. The final test concentration of each compound in the mixture is 150 ppm. The test material is micropipetted (25 ul) into a single well of a 96-well sterile tissue culture plate (COSTAR)[b]and the solvent allowed to evaporate. These "treated" plates are used immediately or stored in a freezer without apparent adverse effects on the compounds.

A freshly prepared volume (50 $\mu$g) of C. elegans in CbMM is micropipetted into each treated well and several control wells per plate. Culture plates are incubated at 20° C.

Observations for efficacy are made under a dissecting microscope at 4, 24 and 48 hours post-immersion. Immediately prior to reading the plate, it is gently tapped to stimulate the movement of the worms. Activity is judged subjectively, but semi-quantitatively, based on the drug effects on motility of the adults and larvae. The criteria are as follows: 9 = worms immobile or dead within 4 hours post immersion in microfilter test solution, 8 = no motility, 7 = markedly reduced motility in approximately 95% of worms, 6 = reduced motility, 5 = slightly reduced motility, 0 = normal motility, same as controls. Other factors indicating activity are easily noted such as death, rigor mortis, contraction, coiling, paralysis, abnormal twitching, reduced worm population in 48 hours and other deviations from normal behavior. Tests results are reported below.

| Test Results Using C. elegans | | |
|---|---|---|
| Compound | Rate ppm | Rating |
| LL-E19020 alpha | 150 | 9 (immobile or dead) |
| LL-E19020 beta | 150 | 9 (immobile or dead) |

## EXAMPLE 12

Trichostrongylus colubriformis/Gerbil Anthelmintic Evaluation

Gerbils are infected with 400 infective larvae of the sheep parasite, T. colubriformis on a day 0. On day 7 gerbils are divided into test and untreated control groups of 2-3 animals. Drug is administered in the feed and are fed from day 7-11. Drugs may also be given by gavage (single oral dose) or injected subcutaneously. Single doses are usually administered on day 7.

Gerbils are killed on day 11 and small intestines removed. The number of T. colubriformis remaining in the intestines of treated animals are counted and compared to the number remaining in the untreated controls. Tests results are reported below.

[a]GIBCO Laboratories, 3175 Staley Road, Grand Island, N.Y. 14027, USA

[b]205 Broadway, Cambridge, MA 02139, USA

| Compound | Dose | % Worms Removed |
|---|---|---|
| LL-E19020 alpha | 250 ppm diet | 56 |
| LL-E19020 beta | 250 ppm diet<br>150 ppm diet<br>62.5 ppm diet | 100<br>86<br>32 |
| LL-E19020 alpha<br>LL-E19020 beta | 10 m/kg single oral dose<br>5 m/kg single oral dose | 23<br>19 |

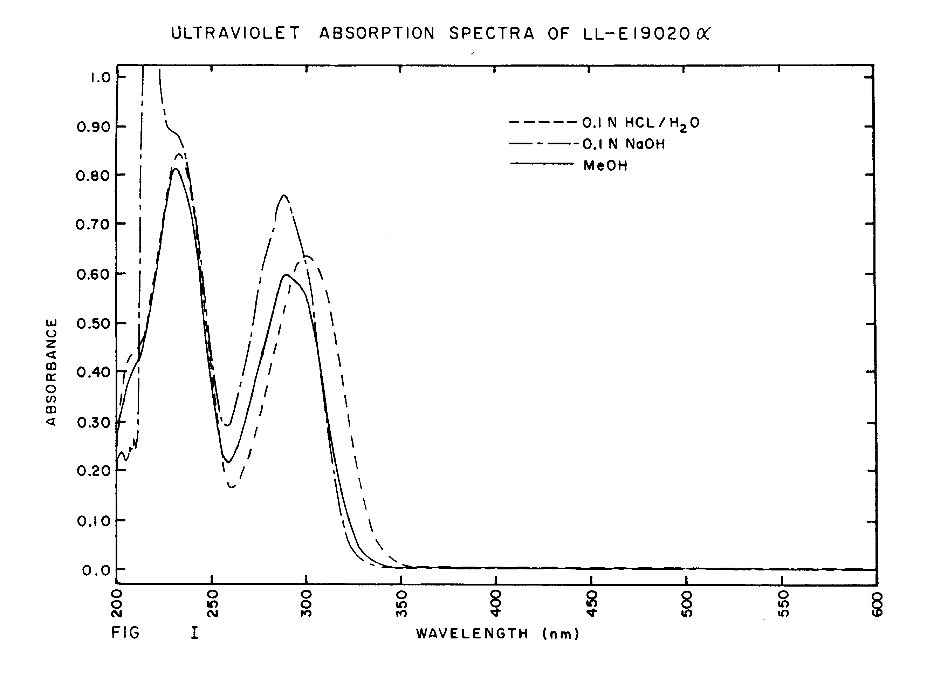

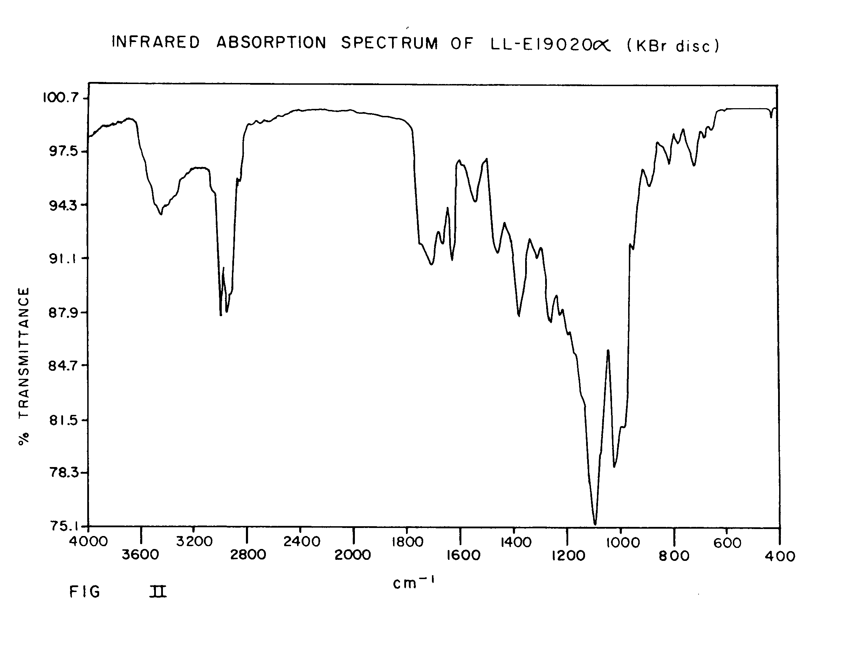

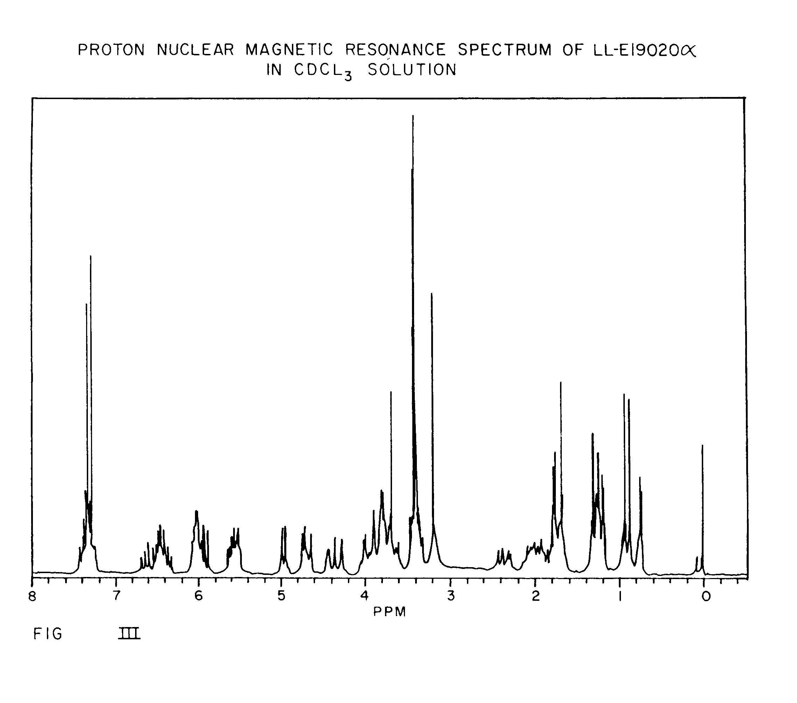

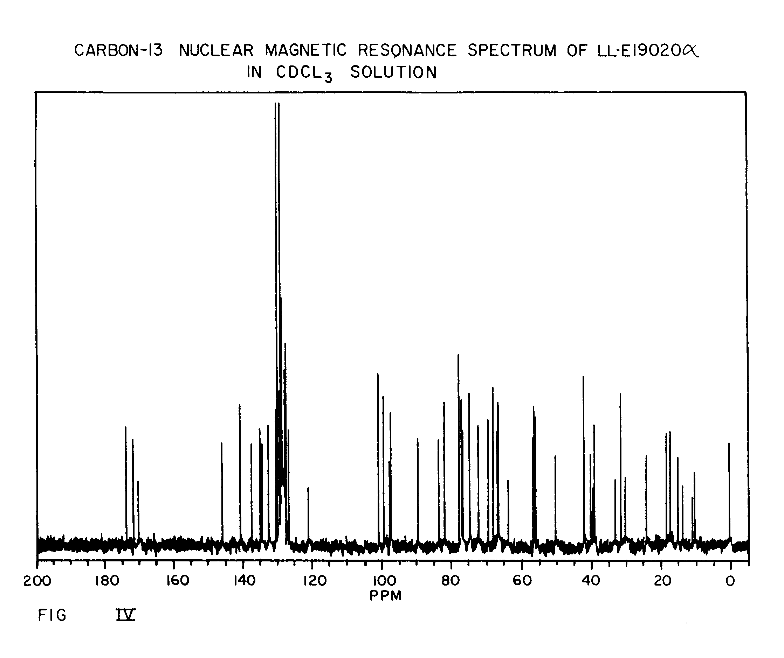

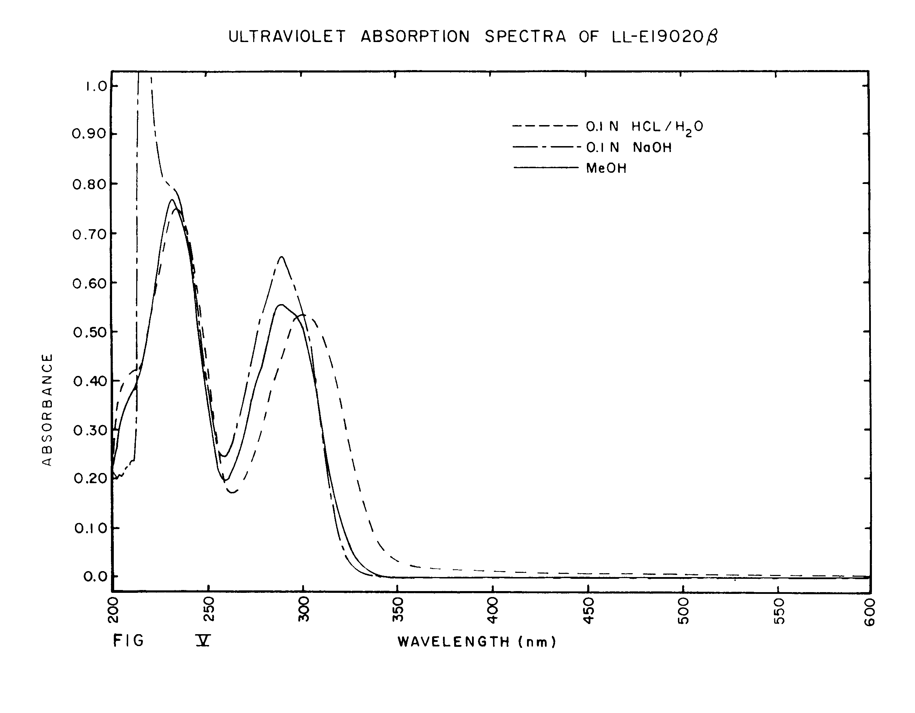

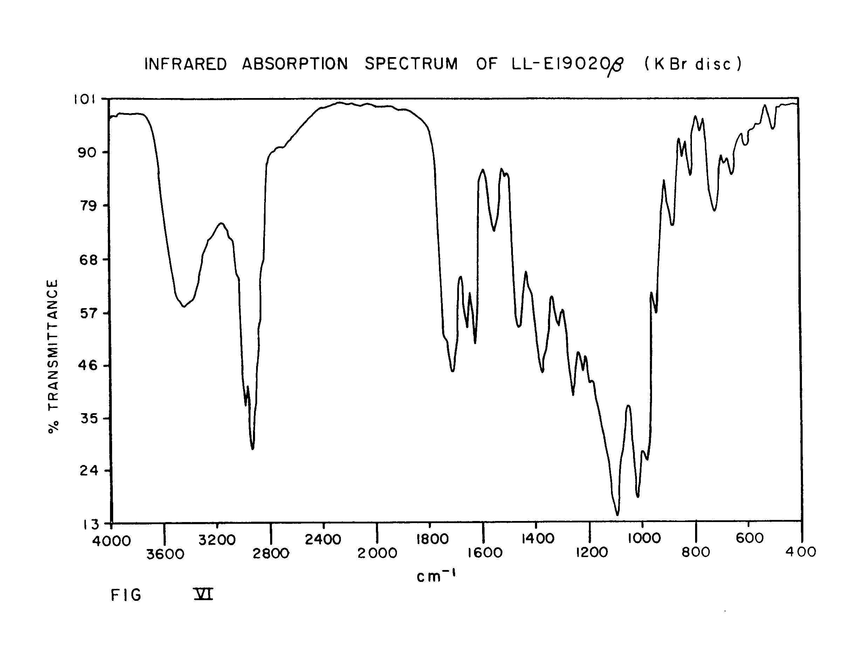

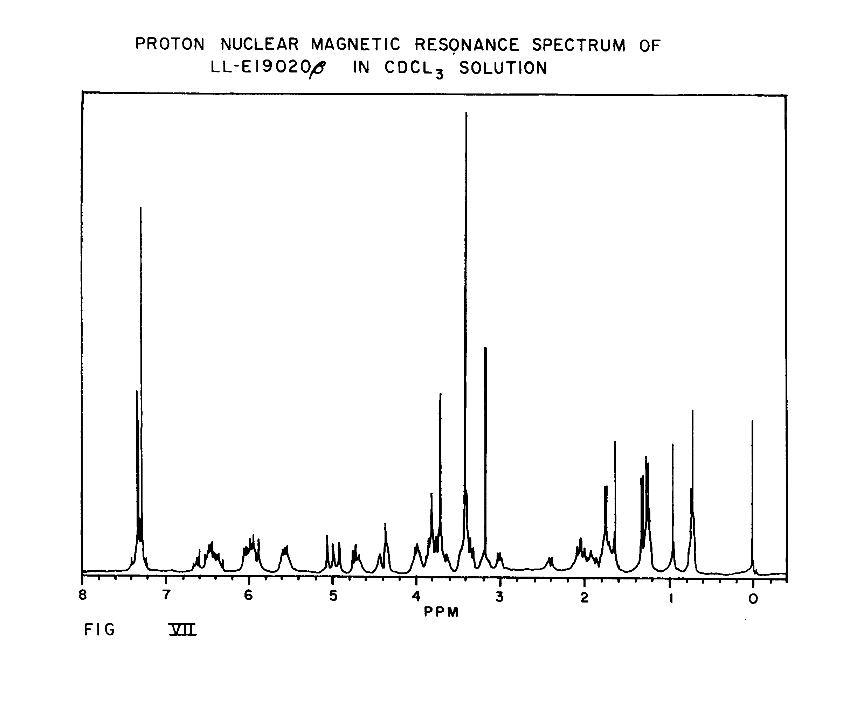

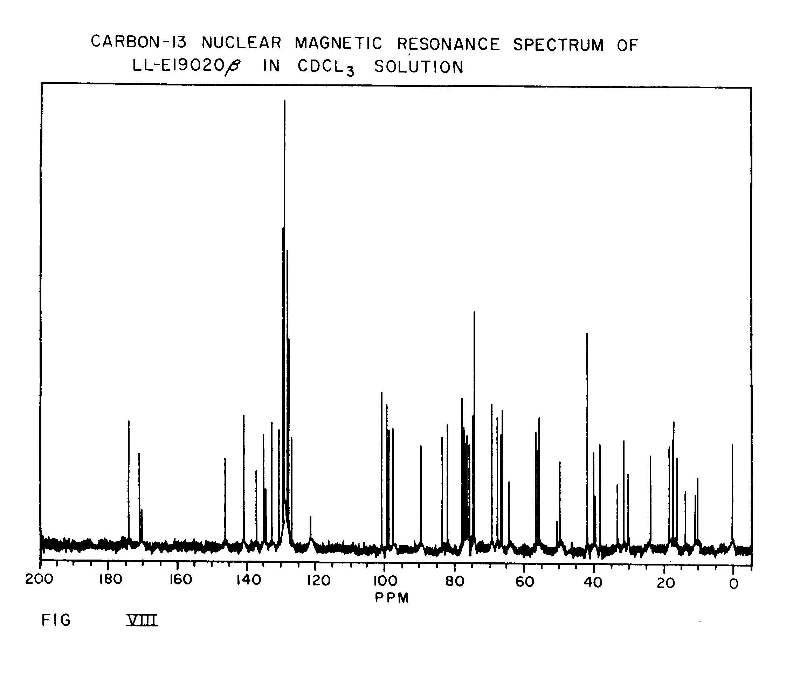

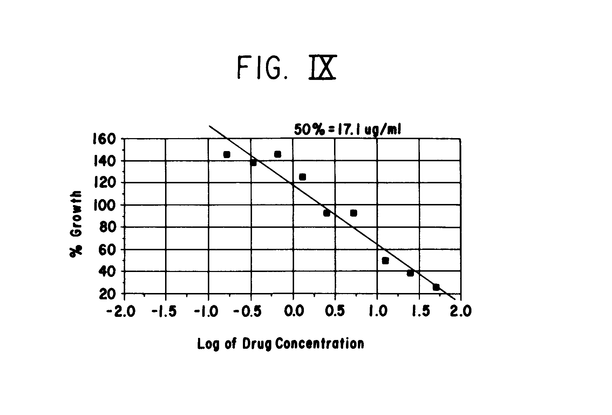

**Claims**
**Claims for the following Contracting States : BE, CH, DE, FR, GB, IT, LI, LU, NL, SE**

1. The compounds designated LL-E19020 alpha and LL-E19020 beta, wherein the compounds have:
   (a) an elemental analysis: C 62.73; H 7.60; N 1.00; O 28.67 (by difference);
   (b) a molecular weight of 1225 (FABMS);
   (c) a specific optical rotation: $[\text{alpha}]_D^{26}$ = 0; (C 0.385, methanol);
   (d) a characteristic ultraviolet absorption spectra as shown in Fig. I of the attached drawings;
   (e) a characteristic infrared absorption spectrum as shown in Fig. II of the attached drawings;
   (f) a characteristic proton nuclear magnetic resonance as shown in Fig. III of the attached drawings; and
   (g) a characteristic carbon-13 nuclear magnetic resonance spectrum as shown in Fig. IV of the attached drawings;
   and
   (a) an elemental analysis: C 63.33; H 7.72; N 1.16; O 27.79 (by difference);
   (b) a molecular weight of 1225 (FABMS);
   (c) a specific optical rotation: $[\text{alpha}]_D^{26}$ = -17±2 (C 0.455, methanol);
   (d) characteristic ultraviolet absorption spectrum as shown in Fig. V of the attached drawings;
   (e) a characteristic infrared absorption spectrum as shown in Fig. VI of the attached drawings;
   (f) a characteristic proton nuclear magnetic resonance spectrum as shown in Fig. VII of the attached drawings; and
   (g) a characteristic carbon-13 nuclear magnetic resonance spectrum as shown in Fig. VIII of the attached drawings,
   respectively.

2. A process for producing antibiotics LL-E19020 alpha and LL-E19020 beta as defined in Claim 1 which comprises aerobically fermenting the organisim Streptomyces lydicus ssp. tanzanius NRRL 18036 or mutants thereof in a liquid medium containing assimilable sources of carbon, nitrogen and inorganic salts, until substantial antibiotic activity is imparted to said medium and then recovering the antibiotics therefrom.

3. A process for producing antibiotics LL-E19020 alpha and LL-E19020 beta as defined in Claim 1 which comprises aerobically fermenting a liquid medium containing assimilable sources of carbon, nitrogen and inorganic salts, which medium has been inoculated with a viable culture of the organism Streptomyces lydicus ssp. tanzanius NRRL 18036 or mutants thereof, maintaining said fermentation culture at a temperature of 25-32°C for a period of about 90-200 hours, harvesting the mash and extracting the antibiotics.

4. A biologically pure culture of the microorganism Streptomyces lydicus ssp. tanzanius, having the identifying characteristics of NRRL 18036, said culture being capable of producing antibiotics LL-E19020 alpha and LL-E19020 beta in recoverable quantities upon fermentation in an aqueous nutrient medium containing assimilable sources of carbon, nitrogen and inorganic anion and cation salts.

5. A composition for controlling or preventing protozoan infections comprising a therapeutically or prophylactically effective amount of the antibiotic LL-E19020 alpha, LL-E19020 beta or a physiologically acceptable salt thereof.

6. A method of increasing the growth rate of meat producing animals and fish, increasing the efficiency of feed utilization and/or improving lactation in lactating ruminents comprising orally or parenterally administering to said animals or fish a growth rate increasing amount of an antibiotic LL-E19020 alpha, LL-E19020 beta or a physiologically-acceptable salt of either antibiotic.

7. An animal feed composition for increasing the growth rate of meat producing animals and fish, for increasing the efficiency of food utilization and/or improving lactation in lactating ruminents comprising an edible animal feed and an effective amount of the antibiotic LL-E19020 alpha, LL-E19020 beta or a physiologically acceptable salt thereof, per ton of animal feed.

8. A composition according to claim 5 for warm-blooded animals containing from about 0.1 ppm to 300 ppm of antibiotic LL-E19020$\alpha$, LL-E19020$\beta$ or a physiologically acceptable salt and the warm-blooded animals are cattle, sheep, swine, goats, horses, poultry or rabbits.

9. A composition for controlling coccidiosis in coccidiosis infected animals comprising a coccidicidally effective amount of the antibiotic LL-E19020$\alpha$, LL-E19020$\beta$ or a physiologically acceptable salt thereof.

10. A composition according to claim 9 containing from about 0.1 ppm to 300 ppm of antibiotic LL-E19020$\alpha$, LL-E19020$\beta$ or a physiologically acceptable salt and the infected animals are poultry, rabbits, cattle, swine and sheep.

11. A composition for controlling protozoan infections in Babesia infected animals comprising a protozoacidally effective amount of the antibiotic LL-E19020$\alpha$, LL-E19020$\beta$ or a physiologically acceptable salt thereof.

12. A composition according to claim 11 for warm-blooded animals containing from about 0.1 ppm to 300 ppm of antibiotic LL-E19020$\alpha$, LL-E19020$\beta$ or a physiologically acceptable salt and the warm-blooded animals are cattle, sheep, dogs or cats.

13. An animal feed composition according to claim 7 for cattle, sheep, goats, swine, or horses containing from about 1 gram to 100 grams per ton of feed of antibiotic LL-E19020$\alpha$, LL-E19020$\beta$ or a physiologically acceptable salt of either antibiotic.

14. A poultry feed composition according to claim 7 containing from about 1.0 gram to 200 grams per ton of feed of antibiotic LL-E19020$\alpha$, LL-E19020$\beta$ or a physiologically acceptable salt of either antibiotic.

**Claims for the following Contracting States : AT, ES, GR**

1. A process for producing the compounds designated LL-E19020 alpha and LL-E19020 beta, wherein the compounds have:
    (a) an elemental analysis: C 62.73; H 7.60; N 1.00; O 23.57 (by difference);
    (b) a molecular weight of 1225 (FABMS);
    (c) a specific optical rotation: $[\text{alpha}]_D^{26} = 0$; (C 0.385, methanol);
    (d) a characteristic ultraviolet absorption spectra as shown in Fig. I of the attached drawings;
    (e) a characteristic infrared absorption spectrum as shown in Fig. II of the attached drawings;
    (f) a characteristic proton nuclear magnetic resonance as shown in Fig. III of the attached drawings; and
    (g) a characteristic carbon-13 nuclear magnetic resonance spectrum as shown in Fig. IV of the attached drawings;
and
    (a) an elemental analysis: C 63.33; H 7.72; N 1.16; O 27.79 (by difference);
    (b) a molecular weight of 1225 (FABMS);
    (c) a specific optical rotation: $[\text{alpha}]_D^{26} = -17\pm2$ (C 0.455, methanol);
    (d) characteristic ultraviolet absorption spectrum as shown in Fig. V or the attached drawings;
    (e) a characteristic infrared absorption spectrum as shown in Fig. VI of the attached drawings;
    (f) a characteristic proton nuclear magnetic resonance spectrum as shown in Fig. VII of the attached drawings; and
    (g) a characteristic carbon-13 nuclear magnetic resonance spectrum as shown in Fig. VIII of the

EP 0 252 380 B1

attached drawings,
respectively, which comprises aerobically fermenting the organism Streptomyces lydicus ssp. tanzanius NRRL 18036 or mutants thereof in a liquid medium containing assimilable sources of carbon, nitrogen and inorganic salts, until substantial antibiotic activity is imparted to said medium and then recovering the antibiotics therefrom.

2. A process for producing antibiotics LL-E19020 alpha and LL-E19020 beta as defined in Claim 1 which comprises aerobically fermenting a liquid medium containing assimilable sources of carbon, nitrogen and inorganic salts, which medium has been inoculated with a viable culture of the organism Stretomyces lydicus ssp. tanzanius NRRL 18036 or mutants thereof, maintaining said fermentation culture at a temperature of 25-32°C for a period of about 90-200 hours, harvesting the mash and extracting the antibiotics.

3. A biologically pure culture of the microorganism Streptomyces lydicus ssp. tanzanius, having the identifying characteristics of NRRL 18036, said culture being capable of producing antibiotics LL-E19020 alpha and LL-E19020 beta in recoverable quantities upon fermentation in an aqueous nutrient medium containing assimilable sources of carbon, nitrogen and inorganic anion and cation salts.

4. A composition for controlling or preventing protozoan infections comprising a therapeutically or prophylactically effective amount of the antibiotic LL-E19020 alpha, LL-E19020 beta or a physiologically acceptable salt thereof.

5. A method of increasing the growth rate of meat producing animals and fish, increasing the efficiency of feed utilization and/or improving lactation in lactating ruminents comprising orally or parenterally administering to said animals or fish a growth rate increasing amount of an antibiotic LL-E19020 alpha, LL-E19020 beta or a physiologically-acceptable salt of either antibiotic.

6. An animal feed composition for increasing the growth rate of meat producing animals and fish, for increasing the efficiency of food utilization and/or improving lactation in lactating ruminents comprising an edible animal feed and an effective amount of the antibiotic LL-E19020 alpha, LL-E19020 beta or a physiologically acceptable salt thereof, per ton of animal feed.

7. A composition according to claim 4 for warm-blooded animals containing from about 0.1 ppm to 300 ppm of antibiotic LL-E19020$\alpha$, LL-E19020$\beta$ or a physiologically acceptable salt and the warm-blooded animals are cattle, sheep, swine, goats, horses, poultry or rabbits.

8. A composition for controlling coccidiosis in coccidiosis infected animals comprising a coccidicidally effective amount of the antibiotic LL-E19020$\alpha$, LL-E19020$\beta$ or a physiologically acceptable salt thereof.

9. A composition according to claim 8 containing from about 0.1 ppm to 300 ppm of antibiotic LL-E19020$\alpha$, LL-E19020$\beta$ or a physiologically acceptable salt and the infected animals are poultry, rabbits, cattle, shine and sheep.

10. A composition for controlling protozoan infections in Babesia infected animals comprising a protozoacidally effective amount of the antibiotic LL-E19020$\alpha$, LL-E19020$\beta$ or a physiologically acceptable salt thereof.

11. A composition-according to claim 10 for warm-blooded animals containing from about 0.1 ppm to 300 ppm of antibiotic LL-E19020$\alpha$, LL-E19020$\beta$ or a physiologically acceptable salt and the warm-blooded animals are cattle, sheep, dogs or cats.

12. An animal feed composition according to claim 6 for cattle, sheep, goats, swine, or horses containing from about 1 gram to 100 grams per ton of feed of antibiotic LL-E19020$\alpha$, LL-E19020$\beta$ or a physiologically acceptable salt of either antibiotic.

13. A poultry feed composition according to claim 6 containing from about 1.0 gram to 200 grams per ton of feed of antibiotic LL-E19020$\alpha$, LL-E19020$\beta$ or a physiologically acceptable salt of either antibiotic.

35

**Revendications**
**Revendications pour les Etats contractants suivants : BE, CH, DE, FR, GB, IT, LI, LU, NL, SE**

1.  Composés désignés par LL-E19020 $\alpha$ et LL-E19020 $\beta$, les composés présentant:

    (a) une analyse élémentaire:
    C 62,73 %; H 7,60 %; N 1,00; O 28,67 % (par différence);
    (b) une masse moléculaire de 1 225 (SM FAB);
    (c) un pouvoir rotatoire spécifique: $[\alpha]_D^{26} = 0$ (c = 0,385, méthanol);
    (d) un spectre d'absorption ultraviolette caractéristique tel que représenté sur la figure I des dessins ci-annexés;
    (e) un spectre d'absorption infrarouge caractéristique tel que représenté sur la figure II des dessins ci-annexés;
    (f) un spectre de résonance magnétique protonique caractéristique tel que représenté sur la figure III des dessins ci-annexés; et
    (g) un spectre de résonance magnétique nucléaire $^{13}$C caractéristique tel que représenté sur la figure LV des dessins ci-annexés;

    et

    (a) une analyse élémentaire:
    C 63,33 %; H 7,72 %; N 1,16 %; O 27,79 % (par différence)
    (b) une masse moléculaire de 1 225 (SM FAB);
    (c) un pouvoir rotatoire spécifique: $[\alpha]_D^{26} = -17\pm2$ (c = 0,455, méthanol);
    (d) un spectre d'absorption ultraviolette caractéristique tel que représenté sur la figure V des dessins ci-annexés;
    (e) un spectre d'absorption infrarouge caractéristique tel que représenté sur la figure VI des dessins ci-annexés;
    (f) un spectre de résonance magnétique protonique caractéristique tel que représenté sur la figure VII des dessins ci-annexés; et
    (g) un spectre de résonance magnétique nucléaire $^{13}$C caractéristique tel que représenté sur la figure VIII des dessins ci-annexés,

    respectivement.

2.  Procédé pour la production des antibiotiques LL-E19020 $\alpha$ et LL-E19020 $\beta$ tels que définis dans la revendication 1, comprenant la culture par fermentation aérobie de l'organisme Streptomyces lydicus ssp. tanzanius NRRL 18036 ou de mutants de celui-ci dans un milieu liquide contenant des sources assimilables de carbone, d'azote et des sels minéraux, jusqu'à ce qu une activité atibiotique notable soit conférée audit milieu, et ensuite la récupération des antibiotiques à partir de celui-ci.

3.  Procédé pour la production des antibiotiques LL-E19020 $\alpha$ et LL-E19020 $\beta$ tels que définis dans la revendication 1, comprenant la fermentation aérobie d'un milieu liquide contenant des sources assimilables de carbone, d'azote et des sels minéraux, lequel milieu étant ensemencé avec une culture viable de l'organisme Streptomyces lydicus ssp. tanzanius NRRL 18036 ou de mutants de celui-ci, le maintien de ladite culture de fermentation à une température de 25 à 32°C pendant une durée d'environ 90-200 heures, la récolte du bouillon de culture et l'extraction des antibiotiques.

4.  Culture biologiquement pure du micro-organisme Streptomyces lydicus ssp. tanzanius ayant les caractéristiques d'identification de NRRL 18036, ladite culture étant capable de produire les antibiotiques LL-E19020 $\alpha$ et LL-E19020 $\beta$ en quantités récupérables après fermentation dans un milieu nutritif aqueux contenant des sources assimilables de carbone, d'azote et des sels d'anions et de cations minéraux.

5.  Composition pour la maîtrise ou la prévention d'infections dues à des protozoaires, comprenant une quantité thérapeutiquement ou prophylactiquement efficace de l'antibiotique LL-E19020 $\alpha$ ou LL-E19020 $\beta$ ou d'un sel physiologiquement acceptable de ceux-ci.

6.  Procédé d'augmentation du taux de croissance d'animaux producteurs de viande et de poissons, d'accroissement de l'efficacité d'utilisation d'aliments et/ou d'amélioration de la lactation chez des ruminants producteurs de lait, comprenant l'administration par voie orale ou parentérale auxdits animaux ou poissons d'une quantité, augmentant le taux de croissance, d'un antibiotique LL-E19020 $\alpha$

ou LL-E19020 $\beta$ ou d'un sel physiologiquement acceptable de l'un ou l'autre antibiotique.

7. Composition d'aliment pour animaux pour l'augmentation du taux de croissance d'animaux producteurs de viande et de poissons, pour l'accroissement de l'efficacité de l'utilisation d'aliments et/ou l'amélioration de la lactation chez des ruminants producteurs de lait, comprenant un aliment comestible pour animaux et une quantité efficace de l'antibiotique LL-E19020 $\alpha$ ou LL-E19020 $\beta$ ou d'un sel physiologiquement acceptable de ceux-ci, par tonne d'aliment pour animaux.

8. Composition selon la revendication 5 pour animaux à sang chaud, contenant d'environ 0,1 ppm à 300 ppm d'antibiotique LL-E19020 $\alpha$ ou LL-E19020 $\beta$ ou d'un sel physiologiquement acceptable de ceux-ci, et les animaux à sang chaud sont des bovins, ovins, porcins, caprins, chevaux, volailles ou lapins.

9. Composition pour la maîtrise de la coccidiose chez des animaux infectés de coccidiose, comprenant une quantité efficace contre la coccidiose de l'antibiotique LL-E19020 $\alpha$ ou LL-E19020 $\beta$ ou d'un sel physiologiquement acceptable de ceux-ci.

10. Composition selon la revendication 9, contenant d'environ 0,1 à 300 ppm de l'antibiotique LL-E19020 $\alpha$ ou LL-E19020 $\beta$ ou d'un sel physiologiquement acceptable de ceuxci, et les animaux infectés sont des volailles, lapins, bovins, porcins et ovins.

11. Composition pour la maîtrise d'infections dues à des protozoaires chez des animaux infectés par Babesia, comprenant une quantité efficace contre les protozoaires de l'antibiotique LL-E19020 $\alpha$ ou LL-E19020 $\beta$ ou d'un sel physiologiquement acceptable de ceux-ci.

12. Composition selon la revendication 11 pour animaux à sang chaud, contenant d'environ 0,1 à 300 ppm de l'antibiotique LL-E19020 $\alpha$ ou LL-E19020 $\beta$ ou d'un sel physiologiquement acceptable de ceux-ci, et les animaux à sang chaud sont des bovins, ovins, chiens ou chats.

13. Composition d'aliment pour animaux selon la revendication 7 pour bovins, ovins, caprins, porcins ou chevaux, contenant d'environ 1 à 100 g par tonne d'aliment de l'antibiotique LL-E19020 $\alpha$ ou LL-E19020 $\beta$ ou d'un sel physiologiquement acceptable de l'un ou l'autre antibiotique.

14. Composition d'aliment pour volailles selon la revendication 7, contenant d'environ 1,0 à 200 g, par tonne d'aliment, d'antibiotique LL-E19020 $\alpha$ ou LL-E19020 $\beta$ ou d'un sel physiologiquement acceptable de l'un ou l'autre antibiotique.

**Revendications pour les Etats contractants suivants : AT, ES, GR**

1. Procédé pour la production des composes désignés par LL-E19020 $\alpha$ et LL-E19020 $\beta$, les composés présentant :
    (a) une analyse élémentaire:
    C 62,73 %; H 7,60 %; N 1,00; O 28,67 % (par différence);
    (b) une masse moléculaire de 1 225 (SM FAB);
    (c) un pouvoir rotatoire spécifique: $[\alpha]_D^{26}$ = 0 (c = 0,385, méthanol);
    (d) un spectre d'absorption ultraviolette caractéristique tel que représenté sur la figure I des dessins ci-annexés;
    (e) un spectre d'absorption infrarouge caractéristique tel que représenté sur la figure II des dessins ci-annexés;
    (f) un spectre de résonance magnétique protonique caractéristique tel que représenté sur la figure III des dessins ci-annexés; et
    (g) un spectre de résonance magnétique nucléaire $^{13}$C caractéristique tel que représenté sur la figure IV des dessins ci-annexés;
    et
    (a) une analyse élémentaire:
    C 63,33 %, H 7,72 %; N 1,16 %; O 27,79 % (par différence)
    (b) une masse moléculaire de 1 225 (SM FAB);
    (c) un pouvoir rotatoire spécifique: $[\alpha]_D^{26}$ = -17±2 (c = 0,455, méthanol);
    (d) un spectre d'absorption ultraviolette caractéristique tel que représenté sur la figure V des dessins ci-annexés;

(e) un spectre d'absorption infrarouge caractéristique tel que représenté sur la figure VI des dessins ci-annexés;

(f) un spectre de resonance magnétique protonique caractéristique tel que représenté sur la figure VII des dessins ci-annexés; et

(g) un spectre de resonance magnétique nucléaire $^{13}$C caractéristique tel que représenté sur la figure VIII des dessins ci-annexés,

respectivement,

procédé comprenant la culture par fermentation aérobie de l'organisme Streptomyces lydicus ssp. tanzanius NRRL 18036 ou de mutants de celui-ci dans un milieu liquide contenant des sources assimilables de carbone, d'azote et des sels minéraux, jusqu'à ce qu'une activité atibiotique notable soit conférée audit milieu, et ensuite la récupération des antibiotiques à partir de celui-ci.

2. Procédé pour la production des antibiotiques LL-E19020 α et LL-E19020 β tels que définis dans la revendication 1, comprenant la fermentation aérobie d'un milieu liquide contenant des sources assimilables de carbone, d'azote et des sels minéraux, lequel milieu étant ensemencé avec une culture viable de l'organisme Streptomyces lydicus ssp. tanzanius NRRL 18036 ou de mutants de celui-ci, le maintien de ladite culture de fermentation à une température de 25 à 32°C pendant une durée d'environ 90-200 heures, la récolte du bouillon de culture et l'extraction des antibiotiques.

3. Culture biologiquement pure du micro-organisme Streptomyces lydicus ssp. tanzanius ayant les caractéristiques d'identification de NRRL 18036, ladite culture étant capable de produire les antibiotiques LL-E19020 α et LL-E19020 β en quantités récupérables après fermentation dans un milieu nutritif aqueux contenant des sources assimilables de carbone, d'azote et des sels d'anions et de cations minéraux.

4. Composition pour la maîtrise ou la prévention d'infections dues à des protozoaires, comprenant une quantité thérapeutiquement ou prophylactiquement efficace de l'antibiotique LL-E19020 α ou LL-E19020 β ou d'un sel physiologiquement acceptable de ceux-ci.

5. Procédé d'augmentation du taux de croissance d'animaux producteurs de viande et de poissons, d'accroissement de l'efficacité d'utilisation d'aliments et/ou d'amélioration de la lactation chez des ruminants producteurs de lait, comprenant l'administration par voie orale ou parentérale auxdits animaux ou poissons d'une quantité, augmentant le taux de croissance, d'un antibiotique LL-E19020 α ou LL-E19020 β ou d'un sel physiologiquement acceptable de l'un ou l'autre antibiotique.

6. Composition d'aliment pour animaux pour l'augmentation du taux de croissance d'animaux producteurs de viande et de poissons, pour l'accroissement de l'efficacité de l'utilisation d'aliments et/ou l'amélioration de la lactation chez des ruminants producteurs de lait, comprenant un aliment comestible pour animaux et une quantité efficace de l'antibiotique LL-E19020 α ou LL-E19020 β ou d'un sel physiologiquement acceptable de ceux-ci, par tonne d'aliment pour animaux.

7. Composition selon la revendication 4 pour animaux à sang chaud, contenant d'environ 0,1 ppm à 300 ppm d'antibiotique LL-E19020 α ou LL-E19020 β ou d'un sel physiologiquement acceptable de ceux-ci, et les animaux à sang chaud sont des bovins, ovins, porcins, caprins, chevaux, volailles ou lapins.

8. Composition pour la maîtrise de la coccidiose chez des animaux infectés de coccidiose, comprenant une quantité efficace contre la coccidiose de l'antibiotique LL-E19020 α ou LL-E19020 β ou d'un sel physiologiquement acceptable de ceux-ci.

9. Composition selon la revendication 8, contenant d'environ 0,1 à 300 ppm de l'antibiotique LL-E19020 α ou LL-E19020 β ou d'un sel physiologiquement acceptable de ceux-ci, et les animaux infectés sont des volailles, lapins, bovins, porcins et ovins.

10. Composition pour la maîtrise d'infections dues à des protozoaires chez des animaux infectés par Babesia, comprenant une quantité efficace contre les protozoaires de l'antibiotique LL-E19020 α ou LL-E19020 β ou d'un sel physiologiquement acceptable de ceux-ci.

**11.** Composition selon la revendication 10 pour animaux à sang chaud, contenant d'environ 0,1 à 300 ppm de l'antibiotique LL-E19020 $\alpha$ ou LL-E19020 $\beta$ ou d'un sel physiologiquement acceptable de ceux-ci, et les animaux à sang chaud sont des bovins, ovins, chiens ou chats.

**12.** Composition d'aliment pour animaux selon la revendication 6 pour bovins, ovins, caprins, porcins ou chevaux, contenant d'environ 1 à 100 g par tonne d'aliment de l'antibiotique LL-E19020 $\alpha$ ou LL-E19020 $\beta$ ou d'un sel physiologiquement acceptable de l'un ou l'autre antibiotique.

**13.** Composition d'aliment pour volailles selon la revendication 6, contenant d'environ 1,0 à 200 g, par tonne d'aliment, d'antibiotique LL-E19020 $\alpha$ ou LL-E19020 $\beta$ ou d'un sel physiologiquement acceptable de l'un ou l'autre antibiotique.

**Patentansprüche**

**Patentansprüche für folgende Vertragsstaaten : BE, CH, DE, FR, GB, IT, LI, LU, NL, SE**

**1.** Verbindungen, die LL-E19020 alpha und LL-E19020 beta bezeichnet werden, mit folgenden Eigenschaften:

(a) eine Elementaranalyse: C 62,73; H 7,60; N 1,00; O 28,67 (durch Differenz);

(b) ein Molekulargewicht von 1225 (FABMS);

(c) eine spezifische optische Drehung:

$[\alpha]_D^{26}$ = 0; (C 0,385, Methanol);

(d) ein charakteristisches Ultraviolett-Absorptionsspektrum wie in Fig. I der beigefügten Zeichnungen gezeigt;

(e) ein charakteristisches Infrarot-Absorptionsspektrum wie in Fig. II der beigefügten Zeichnungen gezeigt;

(f) ein charakteristisches $^1$H-NMR Spektrum wie in Fig. III der beigefügten Zeichnungen gezeigt; und

(g) ein charakteristisches $^{13}$C-NMR Spektrum wie in Fig. IV der beigefügten Zeichnungen gezeigt;

beziehungsweise

(a) eine Elementaranalyse: C 63,33; H 7,72; N 1,16; O 27,79 (durch Differenz);

(b) ein Molekulargewicht von 1225 (FABMS);

(c) eine spezifische optische Drehung:

$[\alpha]_D^{26}$ = -17±2; (C 0,455, Methanol);

(d) ein charakteristisches Ultraviolett-Absorptionsspektrum wie in Fig. V der beigefügten Zeichnungen gezeigt;

(e) ein charakteristisches Infrarot-Absorptionsspektrum wie in Fig. VI der beigefügten Zeichnungen gezeigt;

(f) ein charakteristisches $^1$H-HMR Spektrum wie in Fig. VII der beigefügten Zeichnungen gezeigt; und

(g) ein charakteristisches $^{13}$C-NMR Spektrum wie in Fig. VIII der beigefügten Zeichnungen gezeigt.

**2.** Verfahren zur Herstellung der Antibiotika LL-E19020$\alpha$ und LL-E19020$\beta$, wie in Anspruch 1 definiert, wobei man den Organismus Streptomyces lydicus ssp. tanzanius NRRL 18036 oder Mutanten desselben in einem flüssigen Medium, das assimilierbare Quellen von Kohlenstoff, Stickstoff und anorganischen Salzen enthält, aerobisch fermentiert, bis dem Medium eine wesentliche antibiotische Aktivität verliehen wurde, und anschließend die Antibiotika daraus isoliert.

**3.** Verfahren zur Herstellung der Antibiotika LL-E19020$\alpha$ und LL-E19020$\beta$, wie in Anspruch 1 definiert, wobei man ein flüssiges Medium, das assimilierbare Quellen von Kohlenstoff, Stickstoff und anorganischen Salzen enthält, aerobisch fermentiert, wobei das Medium mit einer lebensfähigen Kultur des Organismus Streptomyces Lydicus ssp. tanzanius NRRL 18036 oder Mutanten desselben geimpft wurde, die Fermentations-Kultur während eines Zeitraums von etwa 90 bis 200 Stunden bei einer Temperatur von 25 bis 32 °C hält, die Maische erntet und die Antibiotika extrahiert.

**4.** Biologisch reine Kultur des Mikroorganismus Streptomyces lydicus ssp. tanzanius mit den kennzeichnenden Merkmalen von NRRL 18036, wobei die Kultur in der Lage ist, die Antibiotika LL-E19020$\alpha$ und LL-E19020$\beta$ bei Fermentierung in einem wässrigen Nährstoffmedium, welches assimilierbare Quellen von Kohlenstoff, Stickstoff und anorganischen Anionen- und Kationen-salzen enthält, in isolierbaren Mengen zu erzeugen.

5. Zusammensetzung zur Bekämpfung oder Verhinderung von Protozoen-Infektionen, umfassend eine therapeutisch oder prophylaktisch wirksame Menge der Antibiotika LL-E19020α, LL-E19020β oder eines physiologisch akzeptablen Salzes derselben.

6. Verfahren zur Steigerung der Wachtumsrate von fleischerzeugenden Tieren und Fischen, zur Steigerung der Effizienz der Futternutzung und/oder zur Verbesserung der Laktation bei laktierenden Wiederkäuern, umfassend die orale oder parenterale Verabreichung einer die Wachtumsrate steigernden Menge eines Antibiotikum LL-E19020α, LL-E19020β oder eines physiologisch akzeptablen Salzes derselben an die Tiere.

7. Tierfutterzusammensetzung zur Steigerung der Wachstumsrate von fleischerzeugenden Tieren und Fischen zur Steigerung der Effizienz der Futternutzung und/oder zur Verbesserung der Laktation bei laktierenden Wiederkäuern, umfassend ein eßbares Tierfutter und eine wirksame Menge der Antibiotika LL-E19020α, LL-E19020β oder eines physiologisch akzeptablen Salzes derselben pro t Tierfutter.

8. Zusammensetzung gemäß Anspruch 5 für warmblütige Tiere, enthaltend etwa 0,1 ppm bis 300 ppm der Antibiotika LL-E19020α, LL-E19020β oder eines physiologisch akzeptablen Salzes, wobei die warmblütigen Tiere Rinder, Schafe, Schweine, Ziegen, Pferde, Geflügel oder Kaninchen sind.

9. Zusammensetzung zur Bekämpfung von Coccidiosis bei coccidiosis infizierten Tieren, umfassend eine zur Bekämpfung von Coccidiosis wirksame Menge der Antibiotika LL-E19020α, LL-E19020β oder eines physiologisch akzeptablen Salzes derselben.

10. Zusammensetzung gemäß Anspruch 9, enthaltend etwa 0,1 ppm bis 300 ppm der Antibiotika LL-E19020α, LL-E19020β oder eines physiologisch akzeptablen Salzes, wobei die infizierten Tiere Geflügel, Kaninchen, Rinder, Schweine und Schafe sind.

11. Zusammensetzung zur Bekämpfung von Protozoen-Infektionen bei mit Babesia infizierten Tieren, umfassend eine zur Protozoenbekämpfung wirksame Menge der Antibiotika LL-E19020α, LL-E19020β oder eines physiologisch akzeptablen Salzes derselben.

12. Zusammensetzung gemäß Anspruch 11 für warmblütige Tiere enthaltend etwa 0,1 ppm bis 300 ppm der Antibiotika LL-E19020α, LL-E19020β oder eines physiologisch akzeptablen Salzes, wobei die warmblütigen Tiere Rinder, Schafe, Hunde oder Katzen sind.

13. Tierfutterzusammensetzung gemäß Anspruch 7 für Rinder, Schafe, Ziegen, Schweine oder Pferde, enthaltend von etwa 1 g bis etwa 100 g der Antibiotika LL-E19020α, LL-E19020β oder eines physiologisch akzeptablen Salzes derselben pro t des Futters.

14. Geflügelfutterzusammensetzung gemäß Anspruch 7, enthaltend von etwa 1,0 g bis 200 g der Antibiotika LL-E19020α, LL-E19020β oder eines physiologisch akzeptablen Salzes derselben pro t des Futters.

**Patentansprüche für folgende Vertragsstaaten : AT, ES, GR**

1. Verfahren zur Herstellung der Verbindungen, die LL-E19020 alpha und LL-E19020 beta bezeichnet werden, mit folgenden Eigenschaften:
    (a) eine Elementaranalyse: C 62,73; H 7,60; N 1,00; O 28,67 (durch Differenz);
    (b) ein Molekulargewicht von 1225 (FABMS);
    (c) eine spezifische optische Drehung:
    $[\alpha]_D^{26}$ = 0; (C 0,385, Methanol);
    (d) ein charakteristisches Ultraviolett-Absorptionsspektrum wie in Fig. I der beigefügten Zeichnungen gezeigt;
    (e) ein charakteristisches Infrarot-Absorptionsspektrum wie in Fig. II der beigefügten Zeichnungen gezeigt;
    (f) ein charakteristisches $^1$H-NMR Spektrum wie in Fig. III der beigefügten Zeichnungen gezeigt; und
    (g) ein charakteristisches $^{13}$C-NMR Spektrum wie in Fig. IV der beigefügten Zeichnungen gezeigt;
    beziehungsweise
    (a) eine Elementaranalyse: C 63,33; H 7,72; N 1,16; O 27,79 (durch Differenz);

EP 0 252 380 B1

(b) ein Molekulargewicht von 1225 (FABMS);

(c) eine spezifische optische Drehung:

$[\alpha]_D^{26}$ = -17±2; (C 0,455, Methanol);

(d) ein charakteristisches Ultraviolett-Absorptionsspektrum wie in Fig. V der beigefügten Zeichnungen gezeigt;

(e) ein charakteristisches Infrarot-Absorptionsspektrum wie in Fig. VI der beigefügten Zeichnungen gezeigt;

(f) ein charakteristisches $^1$H-NMR Spektrum wie in Fig. VII der beigefügten Zeichnungen gezeigt; und

(g) ein charakteristisches $^{13}$C-NMR Spektrum wie in Fig. VIII der beigefügten Zeichnungen gezeigt, wobei man den Organismus Streptomyces lydicus ssp. tanzanius NRRL 18036 oder Mutanten desselben in einem flüssigen Medium, das assimilierbare Quellen von Kohlenstoff, Stickstoff und anorganischen Salzen enthält aerobisch fermentiert, bis dem Medium eine wesentliche antibiotische Aktivität verliehen wurde und anschließend die Antibiotika daraus isoliert.

2. Verfahren zur Herstellung der Antibiotika LL-E19020$\alpha$ und LL-E19020$\beta$, wie in Anspruch 1 definiert, wobei man ein flüssiges Medium, das assimilierbare Quellen von Kohlenstoff, Stickstoff und anorganischen Salzen enthält, aerobisch fermentiert, wobei das Medium mit einer lebensfähigen Kultur des Organismus Streptomyces Lydicus ssp. tanzanius NRRL 18036 oder Mutanten desselben geimpft wurde, die FermentationsKultur während eines Zeitraums von etwa 90 bis 200 Stunden bei einer Temperatur von 25 bis 32 °C hält, die Maische erntet und die Antibiotika extrahiert.

3. Biologisch reine Kultur des Mikroorganismus Streptomyces lydicus ssp. tanzanius mit dem kennzeichnenden Merkmalen von NRRL 18036, wobei die Kultur in der Lage ist, die Antibiotika LL-E19020$\alpha$ und LL-E19020$\beta$ bei Fermentierung in einem wässrigen Nährstoffmedium, welches assimilierbare Quellen von Kohlenstoff, Stickstoff und anorganischen Anionen- und Kationen-salzen enthält, in isolierbaren Mengen zu erzeugen.

4. Zusammensetzung zur Bekämpfung oder Verhinderung von Protozoen-Infektionen, umfassend eine therapeutisch oder prophylaktisch wirksame Menge der Antibiotika LL-E19020$\alpha$, LL-E1 9020$\beta$ oder eines physiologisch akzeptablen Salzes derselben.

5. Verfahren zur Steigerung der Wachtumsrate von fleischerzeugenden Tieren und Fischen, zur Steigerung der Effizienz der Futternutzung und/oder zur Verbessserung der Laktation bei laktierenden Wiederkäuern, umfassend die orale oder parenterale Verabreichung einer die Wachtumsrate steigernden Menge eines Antibiotikum LL-E19020$\alpha$, LL-E19020$\beta$ oder eines physiologisch akzeptablen Salzes derselben an die Tiere.

6. Tierfutterzusammensetzung zur Steigerung der Wachstumsrate von fleischerzeugenden Tieren und Fischen zur Steigerung der Effizienz der Futternutzung und/oder zur Verbesserung der Laktation bei laktierenden Wiederkäuern, umfassend ein eßbares Tierfutter und eine wirksame Menge der Antibiotika LL-E19020$\alpha$, LL-E19020$\beta$ oder eines physiologisch akzeptablen Salzes derselben, pro t Tierfutter.

7. Zusammensetzung gemäß Anspruch 4 für warmblütige Tiere, enthaltend etwa 0,1 ppm bis 300 ppm der Antibiotika LL-E19020$\alpha$, LL-E19020$\beta$ oder eines physiologisch akzeptablen Salzes, wobei die warmblütigen Tiere Rinder, Schafe, Schweine, Ziegen, Pferde, Geflügel oder Kaninchen sind.

8. Zusammensetzung zur Bekämpfung von Coccidiosis bei coccidiosis infizierten Tieren, umfassend eine zur Bekämpfung von Coccidiosis wirksame Menge der Antibiotika LL-E19020$\alpha$, LL-E19020$\beta$ oder eines physiologisch akzeptablen Salzes derselben.

9. Zusammensetzung gemäß Anspruch 8, enthaltend etwa 0,1 ppm bis 300 ppm der Antibiotika LL-E19020$\alpha$, LL-E19020$\beta$ oder eines physiologisch akzeptablen Salzes, wobei die infizierten Tiere Geflügel, Kaninchen, Rinder, Schweine und Schafe sind.

10. Zusammensetzung zur Bekämpfung von Protozoen-Infektionen bei mit Babesia infizierten Tieren, umfassend eine zur Protozoenbekämpfung wirksame Menge der Antibiotika LL-E19020$\alpha$, LL-E19020$\beta$ oder eines physiologisch akzeptablen Salzes derselben.

41

**11.** Zusammensetzung gemäß Anspruch 10 für warmblütige Tiere enthaltend etwa 0,1 ppm bis 300 ppm der Antibiotika LL-E19020α, LL-E19020β oder eines physiologisch akzeptablen Salzes, wobei die warmblütigen Tiere Rinder, Schafe, Hunde oder Katzen sind.

**12.** Tierfutterzusammensetzung gemäß Anspruch 6 für Rinder, Schafe, Ziegen, Schweine oder Pferde, enthaltend von etwa 1 g bis etwa 100 g der Antibiotika LL-E19020α, LL-E19020β oder eines physiologisch akzeptablen Salzes derselben pro t des Futters.

**13.** Geflügelfutterzusammensetzung gemäß Anspruch 6, enthaltend von etwa 1,0 g bis 200 g der Antibiotika LL-E19020α, LL-E19020β oder eines physiologisch akzeptablen Salzes derselben pro t des Futters.

ULTRAVIOLET ABSORPTION SPECTRA OF LL-E19020 $\alpha$

FIG I

INFRARED ABSORPTION SPECTRUM OF LL-E19020α (KBr disc)

FIG  II

cm⁻¹

EP 0 252 380 B1

PROTON NUCLEAR MAGNETIC RESONANCE SPECTRUM OF LL-E19020α
IN CDCL₃ SOLUTION

FIG    III

EP 0 252 380 B1

CARBON-13 NUCLEAR MAGNETIC RESONANCE SPECTRUM OF LL-E19020α
IN CDCL₃ SOLUTION

200  180  160  140  120  100  80  60  40  20  0
PPM

FIG  IV

FIG. V

ULTRAVIOLET ABSORPTION SPECTRA OF LL-E19020β

----- 0.1 N HCL/H₂O
—·— 0.1 N NaOH
——— MeOH

WAVELENGTH (nm)

ABSORBANCE

INFRARED ABSORPTION SPECTRUM OF LL-E19020β (KBr disc)

FIG VI

PROTON NUCLEAR MAGNETIC RESONANCE SPECTRUM OF
LL-E19020β   IN CDCL₃ SOLUTION

PPM

FIG   VII

EP 0 252 380 B1

CARBON-13 NUCLEAR MAGNETIC RESONANCE SPECTRUM OF
LL-E19020β IN CDCL₃ SOLUTION

PPM

FIG    VIII

EP 0 252 380 B1

# FIG. IX